# EUROPEAN PATENT SPECIFICATION

(11) **EP 4 262 391 B1**
(45) Date of publication and mention of the grant of the patent: **03.06.2026**
(21) Application number: 21834826.6
(22) Date of filing: 21.12.2021
(51) Int. Cl.: A01N 37/02, A01N 37/04, A01N 37/06, A01N 37/16, A01N 65/06, A61L 2/18, A61P 31/02, A61P 31/04, A01P 1/00

(54) **A METHOD FOR KILLING, REDUCING THE VIABILITY OF, AND/OR PREVENTING THE GERMINATION OF SPORES OF A BACTERIUM**
VERFAHREN ZUM ABTÖTEN, VERRINGERN DER LEBENSFÄHIGKEIT UND/ODER VERHINDERN DER KEIMUNG VON SPOREN EINES BAKTERIUMS
PROCÉDÉ POUR TUER, RÉDUIRE LA VIABILITÉ ET/OU EMPÊCHER LA GERMINATION DE SPORES D'UNE BACTÉRIE

(30) Priority: 21.12.2020 FI 20206353
(43) Date of publication of application: 25.10.2023
(73) Proprietor: Hankkija Oy, 05800 Hyvinkää (FI); Forchem Oyj, 26820 Rauma (FI)
(72) Inventor: VUORENMAA, Juhani, 05800 Hyvinkää (FI); KETTUNEN, Hannele, 05800 Hyvinkää (FI); ORTE, Juha, 26820 Rauma (FI); RINTOLA, Mikko, deceased (ZZ); MCGRATH, JOHN, Belfast, BT7 1NN (GB); FAIRLEY, Derek, Belfast, BT7 1NN (GB); MANESIOTIS, Panagiotis, Belfast, BT7 1NN (GB); BELL, Stephen, Belfast, BT7 1NN (GB); CONNOR, Mairead, Belfast, BT12 6BA (GB)
(74) Representative: Papula Oy
(86) International application number: PCT/FI2021/050902
(87) International publication number: WO 2022/136737

(56) References cited:
- WO-A1-2013/171370
- WO-A1-2014/184430
- US-A1- 2007 053 850
- US-A1- 2013 045 954
- US-B2- 6 770 147
- BROWN K L: "Control of bacterial spores", BRITISH MEDICAL BULLETIN., vol. 56, no. 1, 1 January 2000 (2000-01-01), GB, pages 158 - 171, XP093227379, ISSN: 0007-1420, DOI: 10.1258/0007142001902860
- SETLOW PETER ET AL: "What's new and notable in bacterial spore killing!", WORLD JOURNAL OF MICROBIOLOGY AND BIOTECHNOLOGY, vol. 37, no. 8, 1 August 2021 (2021-08-01), Dordrecht, XP093227380, ISSN: 0959-3993, Retrieved from the Internet <URL:https://link.springer.com/article/10.1007/s11274-021-03108-0/fulltext.html> DOI: 10.1007/s11274-021-03108-0
- STRASSMANN JOAN E.: "Social Evolution: Early Production of Deadly Males by Competing Queens", CURRENT BIOLOGY, vol. 16, no. 24, 1 December 2006 (2006-12-01), GB, pages R1023 - R1025, XP093227381, ISSN: 0960-9822, DOI: 10.1016/j.cub.2006.11.014
- CONNOR MAIRÉAD ET AL: "Evolutionary clade affects resistance of Clostridium difficile spores to Cold Atmospheric Plasma", SCIENTIFIC REPORTS, vol. 7, no. 1, 3 February 2017 (2017-02-03), US, XP093227382, ISSN: 2045-2322, Retrieved from the Internet <URL:https://www.nature.com/articles/srep41814> DOI: 10.1038/srep41814
- KOHOUT CORDULA K ET AL: "Bacterial growth dynamics and corresponding metabolite levels in the extraction area of an Austrian sugar beet factory using antimicrobial treatment", JOURNAL OF THE SCIENCE OF FOOD AND AGRICULTURE, vol. 100, no. 6, 11 February 2020 (2020-02-11), GB, pages 2713 - 2721, XP093227378, ISSN: 0022-5142, Retrieved from the Internet <URL:https://onlinelibrary.wiley.com/doi/full-xml/10.1002/jsfa.10303> DOI: 10.1002/jsfa.10303
- DYAS A ET AL: "The activity of glutaraldehyde against Clostridiurn difticile", JOURNAL OF HOSPITAL INFECTION, 1 January 1985 (1985-01-01), pages 41 - 456, XP093267769
- BELL STEPHEN ET AL: "Rosin as a natural alternative for the effective disinfection of ESKAPE pathogens and Clostridioides difficile spores", POTENTIAL OF BULB-ASSOCIATED BACTERIA FOR BIOCONTROL OF HYACINTH SOFT ROT CAUSED BY DICKEYA ZEAE., vol. 135, no. 1, 2 January 2024 (2024-01-02), GB, XP093267771, ISSN: 1365-2672, Retrieved from the Internet <URL:https://academic.oup.com/jambio/article-pdf/135/1/lxae008/56416992/lxae008.pdf> DOI: 10.1093/jambio/lxae008
- SAVLUCHINSKE-FEIO SONIA ET AL: "Antimicrobial activity of resin acid derivatives", APPLIED MICROBIOLOGY AND BIOTECHNOLOGY, SPRINGER BERLIN HEIDELBERG, BERLIN/HEIDELBERG, vol. 72, no. 3, 5 August 2006 (2006-08-05), pages 430 - 436, XP037015924, ISSN: 0175-7598, [retrieved on 20060805], DOI: 10.1007/S00253-006-0517-0
- SIPPONEN A ET AL: "Antimicrobial properties of natural coniferous rosin in the European Pharmacopoeia challenge test.", APMIS, vol. 119, 1 January 2011 (2011-01-01), pages 720 - 724, XP055821038

## Description

### TECHNICAL FIELD

The present disclosure relates to a method for killing, reducing the viability of, and/or preventing the germination of spores of a spore-forming bacterium.

### BACKGROUND

Spores of spore-forming bacteria are typically much more resistant to antimicrobial agents, biocides, disinfectants and/or sanitizers than vegetative forms of the bacteria. For example, the spores of the clinically important bacterium *Clostridium difficile* are notoriously difficult to inactivate and/or inhibit. While C. *difficile* is pathogenic in the vegetative state, infection by a spore could precede it. Similar considerations relate to other dormant stage forms of bacteria.

The life-cycle of a spore-forming bacterium involves a complex regulatory network which promotes vegetative cell growth followed by dormant spore formation and back again via the processes of sporulation, germination and outgrowth. An effective sporicidal treatment should disrupt this life cycle and achieve a loss of the spore's ability to germinate and grow or possibly even kill spores.

Microbial susceptibilities to antimicrobial agents, biocides, disinfectants and/or sanitizers also differ greatly. At present, the structures of bacterial and other dormant stage forms, such as spores, are not yet fully understood, and there are considerable differences in the structures and compositions of bacterial dormant stage forms and vegetative cells. It may therefore be challenging or even impossible to predict which agents would be effective against such dormant stage forms, or against which species, and to find agents having relatively broad activities.

A number of sporicidal products are commercially available. Many of these products have significant drawbacks in terms of operator safety, efficacy, cost and shelf life. For example, sodium hypochlorite, a chlorine-releasing compound that is a constituent of many commercially available bleach products, can pose a health risk to workers: free chlorine can cause severe irritation to eyes and throat.

US 2007/0053850 discloses aqueous disinfectant compositions.

Savluchinske-Feio et al., Appl Microbiol Biotechnol 2006, 72, 430-436 describes antimicrobial activity of resin acid derivatives.

Sipponen and Laitinen, APMIS 2011, 119, 720-724 describes antimicrobial properties of natural coniferous rosin in the European Pharmacopoeia challenge test.

WO 2013/171370 discloses modified tall oil fatty acid/feed supplement for use in the prevention of growth of harmful bacteria in the animal digestive tract.

WO 2014/184430 discloses tall oil fatty acid for use in the prevention of growth of harmful bacteria in the animal digestive tract.

Brown, British Medical Bulletin 2000, 56, 158-171 mentions that bacterial spores are much more resistant than their vegetative counterparts.

Setlow et al., World Journal of Microbiology and Biotechnology 2021, 37, 144 reviews research on bacterial spore killing.

Lizunov and Zimmerberg, Current Biology 16, 24, R1026 mentions that endospores are resistant to many agents used to kill bacteria.

Connor et al., Scientific Reports 2017, 7:41814 describes exposure of *C. difficile* spores to non-thermal atmospheric pressure gas plasma.

US 2013/045954 discloses antimicrobial compositions comprising a terpenoid in combination with an antimicrobial agent.

US 6,770,147 describes a method for producing sugar or sugar-containing products from sugar-containing vegetable raw materials in the presence of added natural food-compatible resins.

Kohout et al, Journal of the Science of Food and Agriculture 2020, 100, 6, 2713-2721 describes bacterial growth dynamics and corresponding metabolite levels in the extraction area of an Austrian sugar beet factory using antimicrobial treatment.

### SUMMARY

A non-therapeutic method for killing, reducing the viability of, and/or preventing the germination of spores of a spore-forming bacterium is disclosed. The method may comprise applying a composition comprising one or more resin acids to the spores of the bacterium, thereby killing, reducing the viability of, and/or preventing the germination of the spores of the bacterium at least partially.

Non-therapeutic use of a composition comprising one or more resin acids for killing, reducing the viability of, and/or preventing the germination of spores of a spore-forming bacterium is also disclosed. The composition is applied to the spores of the bacterium.

### BRIEF DESCRIPTION OF THE DRAWINGS

The accompanying drawings, which are included to provide a further understanding of the embodiments and constitute a part of this specification, illustrate various embodiments. In the drawings:
Figures 1A to 1E show the effect of Rosin soap (5 % w/v) against five clades of *Clostridium difficile,* A) R20291, B) M120, C) CF5, D) CD305, E) TL178 at 0, 4 and 8 minutes.
Figures 2A to 2E illustrate the effect of rosin-fatty acid (DTO) soap (5 % v/v) against five clades of *Clostridium difficile,* A) R20291, B) M120, C) CF5, D) CD305, E) TL178 at 0, 4 and 8 minutes.
Figures 3A to 3E show the effect of Finnish Tall Rosin (5 % w/v) against five clades of *Clostridium difficile,* A) R20291, B) M120, C) CF5, D) CD305, E) TL178 at 0, 4 and 8 minutes.
Figures 4A to 4E show the effect of Indonesian Gum Rosin (5 % w/v) against five clades of *Clostridium difficile,* A) R20291, B) M120, C) CF5, D) CD305, E) TL178 at 0, 4 and 8 minutes.
Figures 5A to 5E illustrate the effect of purified rosin (5 % w/v) against five clades of *Clostridium difficile,* A) R20291, B) M120, C) CF5, D) CD305, E) TL178 at 0, 4 and 8 minutes.
Figures 6A to 6E show the effect of Rosin Soap (2.5 % w/v) against five clades of *Clostridium difficile,* A) R20291, B) M120, C) CF5, D) CD305, E) TL178 at 0 and 8 minutes.
Figures 7A to 7E show the effect of rosin-fatty acid (DTO) soap (2.5 % v/v) against five clades of *Clostridium difficile,* A) R20291, B) M120, C) CF5, D) CD305, E) TL178 at 0 and 8 minutes.
Figures 8A to 8E show the effect of Finnish Tall Oil (2.5 % w/v) against five clades of *Clostridium difficile,* A) R20291, B) M120, C) CF5, D) CD305, E) TL178 at 0 and 8 minutes.
Figures 9A to 9E show the effect of Indonesian Gum Rosin (2.5 % w/v) against five clades of *Clostridium difficile,* A) R20291, B) M120, C) CF5, D) CD305, E) TL178 at 0 and 8 minutes.
Figures 10A to 10E show the effect of purified rosin (2.5 % w/v) against five clades of *Clostridium difficile,* A) R20291, B) M120, C) CF5, D) CD305, E) TL178 at 0 and 8 minutes.
Figures 11A to 11E show the effect of Rosin Soap (1 % w/v) against five clades of *Clostridium difficile,* A) R20291, B) M120, C) CF5, D) CD305, E) TL178 at 0 and 8 minutes.
Figures 12A to 12E show the effect of rosin-fatty acid (DTO) soap (1 % v/v) against five clades of *Clostridium difficile,* A) R20291, B) M120, C) CF5, D) CD305, E) TL178 at 0 and 8 minutes.
Figures 13A to 13E show the effect of Finnish Tall Oil (1 % w/v) against five clades of *Clostridium difficile,* A) R20291, B) M120, C) CF5, D) CD305, E) TL178 at 0 and 8 minutes.
Figures 14A to 14E illustrate the effect of Indonesian Gum Rosin (1 % w/v) against five clades of *Clostridium difficile,* A) R20291, B) M120, C) CF5, D) CD305, E) TL178 at 0 and 8 minutes.
Figures 15A to 15E show the effect of purified rosin (1 % w/v) against five clades of *Clostridium difficile,* a) R20291, b) M120, c) CF5, d) CD305, e) TL178 at 0 and 8 minutes.
Figs. 16 to 29 do not fall within the scope of the invention.

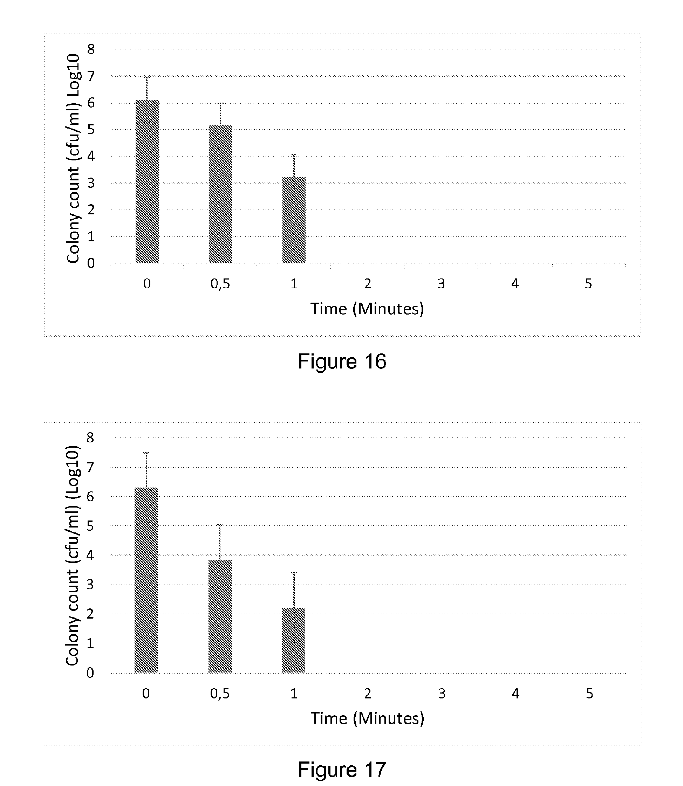

Fig. 16 shows the effect of Rosin soap (5% w/v) after 0, 0.5, 1, 2 ,3, 4 and 5mins exposure against the ESKAPE pathogen *Acinetobacter baumannii.*
Fig. 17 shows the effect of Rosin soap (5% w/v) after 0, 0.5, 1, 2, 3, 4 and 5mins exposure against the ESKAPE pathogen *Enterococcus faecium.*

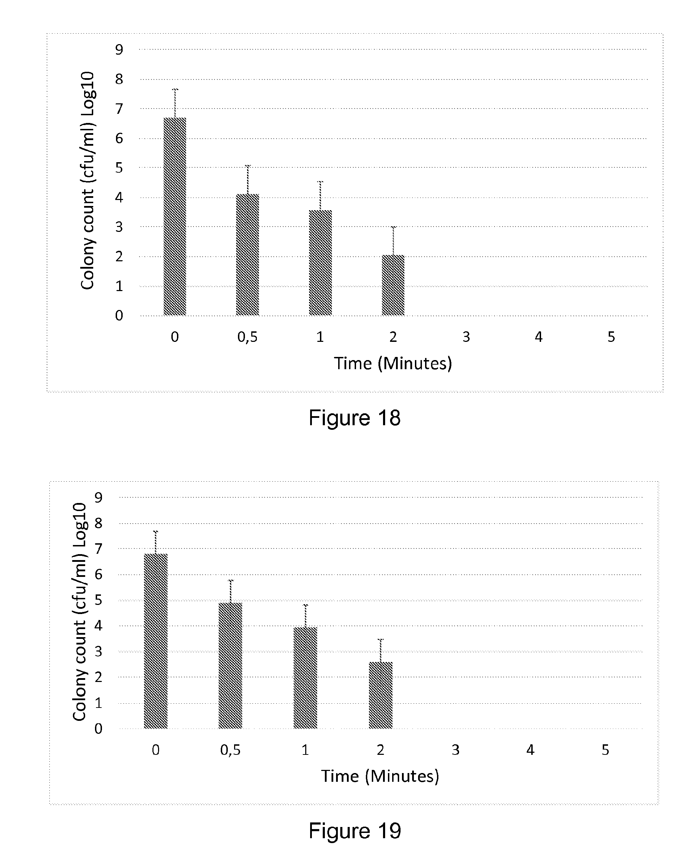

Fig. 18 shows the effect of Rosin soap (5% w/v) after 0, 0.5, 1, 2, 3, 4 and 5mins exposure against the ESKAPE pathogen *Methicillin-* resistant *Staphylococcus aureus.*

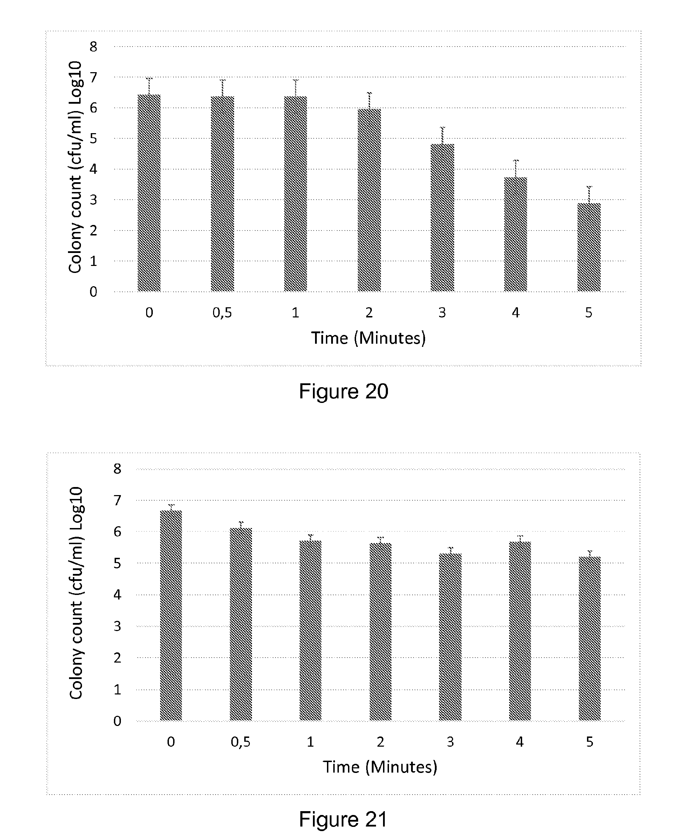

Fig. 19 demonstrates the effect of Rosin soap (5% w/v) after 0, 0.5, 1, 2, 3, 4 and 5mins exposure against the ESKAPE pathogen *Pseudomonas aeruginosa.* Fig. 20 shows the effect of Rosin soap (5% w/v) after 0, 0.5, 1, 2, 3, 4 and 5mins exposure against the ESKAPE pathogen *Enterobacter cloacae.*
Fig. 21 shows the effect of Rosin soap (5% w/v) after 0, 0.5, 1, 2, 3, 4 and 5mins exposure against the ESKAPE pathogen *Klebsiella pneumoniae.*

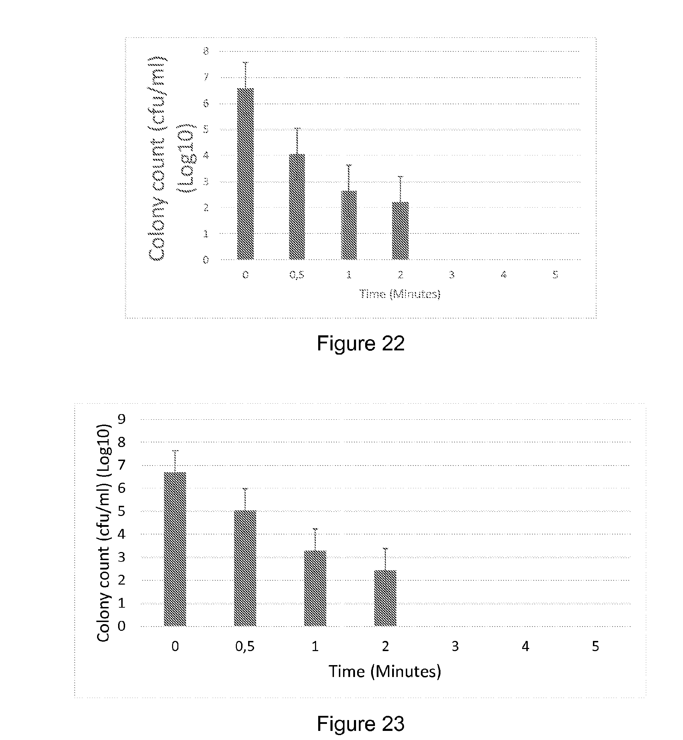

Fig. 22 shows the effect of Rosin soap (5 % w/v) in the presence of 0.6% bovine serum albumin after 0, 0.5, 1, 2, 3, 4 and 5mins exposure against the ESKAPE pathogen *Enterococcus faecium.*
Fig. 23 illustrates the effect of Rosin soap (5 % w/v) in the presence of 0.6% bovine serum albumin after 0, 0.5, 1, 2, 3, 4 and 5mins exposure against the ESKAPE pathogen *Acinetobacter baumannii.*

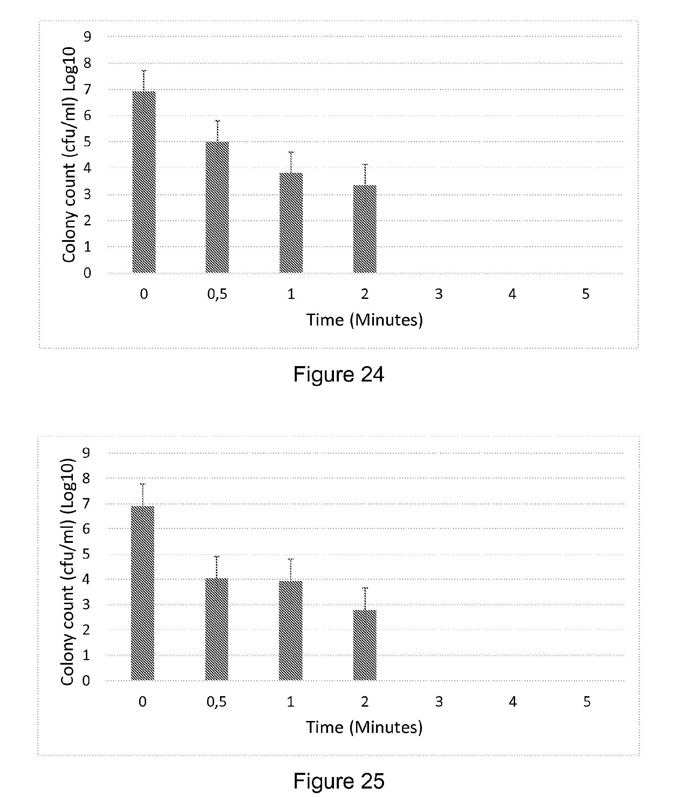

Fig. 24 shows the effect of Rosin soap (5 % w/v) in the presence of 0.6% bovine serum albumin after 0, 0.5, 1, 2, 3, 4 and 5mins exposure against the ESKAPE pathogen *Pseudomonas aeruginosa.*
Fig. 25 demonstrates the effect of Rosin soap (5 % w/v) in the presence of 0.6% bovine serum albumin after 0, 0.5, 1, 2, 3, 4 and 5mins exposure against the ESKAPE pathogen *Methicillin-*resistant *Staphylococcus aureus.*

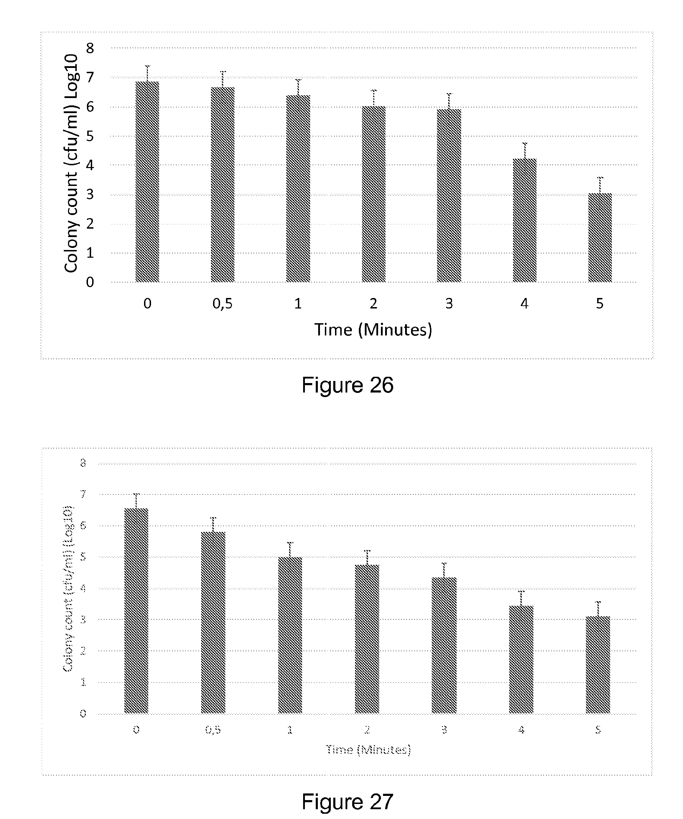

Fig. 26 shows the effect of Rosin soap (5 % w/v) in the presence of 0.6% bovine serum albumin after 0, 0.5, 1, 2, 3, 4 and 5mins exposure against the ESKAPE pathogen *Enterobacter cloacae.*
Fig. 27 shows the effect of Rosin soap (5 % w/v) in the presence of 0.6% bovine serum albumin after 0, 0.5, 1, 2, 3, 4 and 5mins exposure against the ESKAPE pathogen *Klebsiella pneumoniae.*

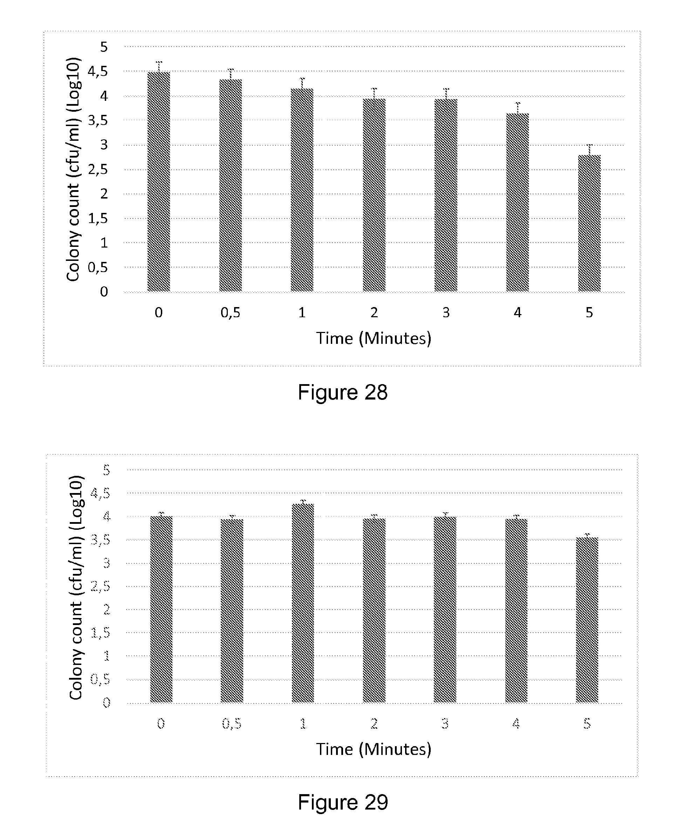

Figure 28 illustrates the effect of Rosin soap (5% w/v) after 0, 0.5, 1, 2, 3, 4 and 5mins exposure against *Candida albicans.*
Figure 29 shows the effect of Rosin soap (5 % w/v) in the presence of 0.6% bovine serum albumin after 0, 0.5, 1, 2, 3, 4 and 5mins exposure against *Candida albicans.*

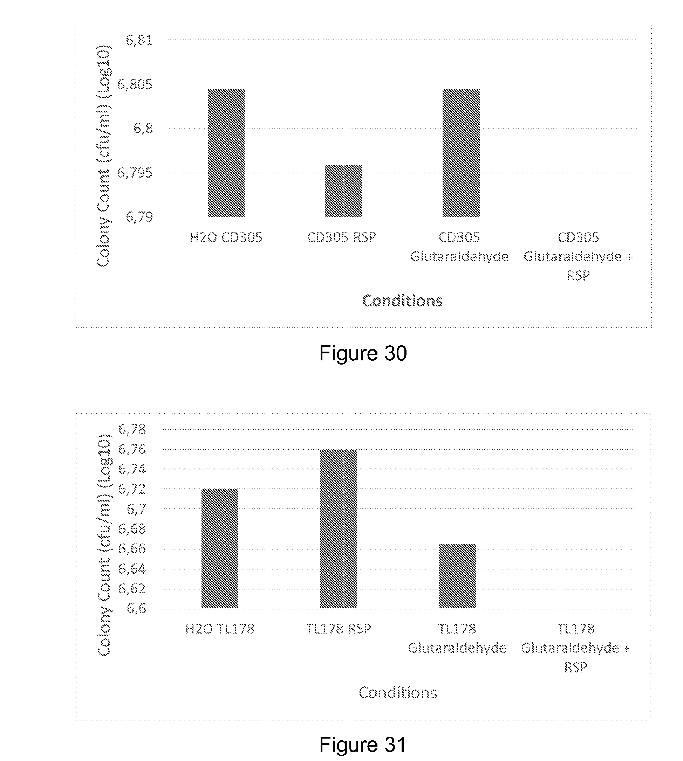

Figures 30 and 31 show the effect of Rosin Soap Powder (2.5 % w/v) and 0.5 % glutaraldehyde, 10 minutes exposure against *Clostridium difficile,* strain CD305 and TL178, respectively. Results are the means of two biological and two technical replicates.

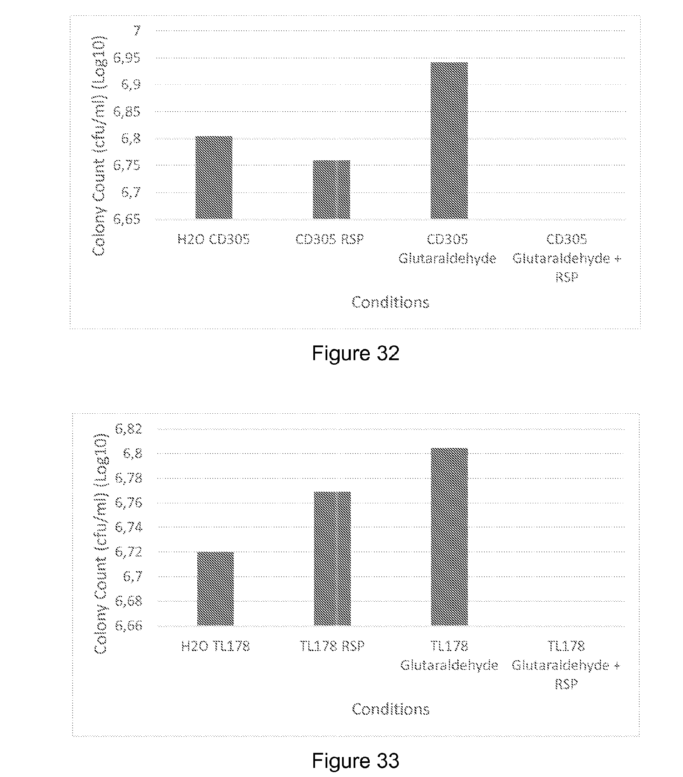

Figures 32 and 33 show the effect of Rosetax-21 (2.5 % w/v) and 0.5 % glutaraldehyde 10 minutes exposure against *Clostridium difficile,* strain CD305 and TL178, respectively. Results are the means of two biological and two technical replicates.

### DETAILED DESCRIPTION

A method for killing, reducing the viability of, and/or preventing germination of dormant stage forms of a bacterium capable of forming the dormant stage forms is disclosed.

The method comprises applying a composition comprising one or more resin acids to the dormant stage forms of the bacterium, thereby killing, reducing the viability of, and/or preventing the germination of the dormant stage forms of the bacterium at least partially.

Use of a composition comprising one or more resin acids for killing, reducing the viability of, and/or preventing the germination of dormant stage forms of a bacterium capable of forming the dormant stage forms is also disclosed.

Any embodiments described in this specification may be understood as relating to the method, to the use, or to both.

Compositions comprising one or more resin acids may be capable of significantly decreasing the germination of dormant stage forms of bacteria capable of forming the dormant stage forms, for example up to 98 %. The effect may be observed across broad concentration ranges of the one or more resin acids and exposure times.

Further, the compositions may also be effective across various other bacterial genera and species, including various clinically important hospital pathogens and other commercially important species, including both Gram-positive and Gram-negative bacteria.

Compositions comprising one or more resin acids are also relatively non-toxic and safe for use.

The composition may kill, reduce the viability of, and/or prevent the germination of the dormant stage forms of the bacterium at least partially.

For example, the composition may prevent the germination of at least 50 %, or at least 95 %, or at least 97 %, of the dormant stage forms of the bacterium. For example, the composition may kill at least 50 %, or at least 95 %, or at least 97 %, of the dormant stage forms of the bacterium. For example, the composition may reduce the viability of at least 50 %, or at least 95 %, or at least 97 %, of the dormant stage forms of the bacterium.

The efficacy may depend e.g. on the time of exposure of the dormant stage forms to the composition. For example, the composition may prevent the germination of at least 50 %, or at least 95 %, or at least 97 %, of the dormant stage forms of the bacterium at 0 - 8 minutes. For example, the composition may kill at least 50 %, or at least 95 %, or at least 97 %, of the dormant stage forms of the bacterium at 0 - 8 minutes. For example, the composition may reduce the viability of at least 50 %, or at least 95 %, or at least 97 %, of the dormant stage forms of the bacterium at 0 - 8 minutes.

The efficacy, i.e. the proportion of the dormant stage forms which the composition is capable of preventing the germination of, or killing, or reducing the viability of, may be measured using known methods. A method of determining the efficacy is described in the Examples of the present specification. For example, the efficacy may be determined by suspending the dormant stage forms, such that the dormant stage form concentration is known, to the composition; incubating the suspension for a time period (for example, 0 (control) and 8 minutes); serially diluting the incubated composition e.g. in deionized water; and culturing the dilution series of the composition in a medium suitable for the germination and growth of the bacterium.

In the context of this specification, the term "dormant stage form" may be understood as referring to a resting stage of a bacterium, that may allow the bacterium to survive in unfavourable environmental conditions. Such dormant stage forms may be metabolically quiescent (i.e. the metabolic processes of the dormant stage form are slowed as compared to vegetative form(s) of the bacterium), stress resistant, and/or capable of propagation (germination) in response to suitable growth conditions.

The dormant stage forms are spores, and the bacterium is a spore-forming bacterium. In other words, the method is a method for killing, reducing the viability of, and/or preventing the germination of spores of a spore-forming bacterium.

The bacterium may be a Gram-positive bacterium.

The bacterium may be a bacterium of the genus *Bacillus* and/or *Clostridium.*

The bacterium may be a bacterium of the species *Clostridium difficile* and/or *Clostridium perfringens.*

While not falling within the scope of the invention, the composition may additionally be used for and/or capable of killing and/or reducing the viability of other bacteria. For example, the composition may additionally be used for and/or capable of killing and/or reducing the viability of species such as the so-called ESKAPE pathogens *(Enterococcus pneumoniae, Staphylococcus aureus, Klebsiella pneumoniae, Acinetobacter baumannii, Pseudomonas aeruginosa* and/or *Enterobacter* spp.). ESKAPE pathogens often cause hospital-acquired infections. The ability of a composition to kill and/or reduce the viability of ESKAPE pathogens may indicate that the composition is a broad-spectrum composition.

The composition may comprise or be at least one of tall oil rosin (TOR), wood rosin, gum rosin, distilled tall oil (DTO), wood extractives, oleoresins, or any mixture or combination thereof. These substances contain resin acids.

The composition may consist of one or more resin acids selected from tall oil rosin (TOR), wood rosin, gum rosin, distilled tall oil (DTO), wood extractives, oleoresins, and any mixture or combination thereof.

Resin acids are present in various tree species in most softwoods, and some hardwoods and most commonly in coniferous types of trees like spruce and pine species where different resin acids can be found and obtained. Some amounts may also be extracted from the forest-based biomass materials, such as from peat. Industrially resin acids containing wood extractives (rosin) may be mainly produced by kraft pulp processing to obtain tall oil products such as crude tall oil (CTO), tall oil pitch (TOP), tall oil rosin (TOR), tall oil fatty acid (TOFA) and distilled tall oil (DTO) or by wounding and tapping trees (GUM rosin) or extracting/distillating wood based materials in different ways (wood rosin).

Fractional distillation of crude tall oil (CTO), obtained as a by-product of the Kraft process of wood pulp manufacture, may produce depitched tall oil, which typically comprises more than 10 % (w/w) of resin acids and less than 90 % (w/w) of fatty acids. CTO may be obtained via acidulation of crude tall oil soap or crude sulphate soap (TOS). TOS may be separated from cooking liquid in pulp mill often called black liqueur during pulping process. Further refinement of depitched tall oil may produce turpentine, tall oil fatty acid (TOFA), distilled tall oil (DTO) and tall oil rosin (TOR), or mixtures of these, which may be available in a variety of compositions differing in their fatty acids and resin acids content.

TOFA may also be contemplated, for example concentrated TOFA, which may comprise e.g. 10 % (w/w) of resin acids.

TOR may be a resin acid fraction separated by vacuum distillation from crude tall oil (CTO). Wood rosin may be a fraction separated by steam distillation or other means from dead trees, tree stumps, branches etc. Gum rosin may be a resin fraction obtainable by steam distillation or separation by other means from resin harvested (often referred to as tapping) from a living tree.

Gum rosin (also referred to as gum resin) is widely produced in China, Indonesia and Brazil. Wood rosin mainly comes from the USA. TOR is produced in the USA and Scandinavia and to a lesser extent in Central Europe, New Zealand and Russia. Substances containing resin acid and obtained by vacuum distillation from crude tall oil include distilled tall oil (DTO), tall oil fatty acid (TOFA) and tall oil pitch (TOP). DTO may contain about 10 - 40 % (w/w) of resin acids. CTO may typically contain about 15 - 70 % (w/w) of resin acids. The lowest resin acid contents may generally be provided by the cooking of mixed wood pulp.

In an embodiment, the composition comprises TOR.

The term "tall oil rosin" or "TOR" may be understood as referring to a composition obtainable or obtained by distillation of crude tall oil and further refinement of distilled tall oil. TOR may comprise 60 - 99 % (w/w) of resin acids, or typically (for example, in typical commercial preparations) 60 - 94 % (w/w).

TOR may comprise at least 60 % (w/w) of one or more resin acids. In one embodiment, the TOR comprises at least 85 % (w/w) of one or more resin acids. An example of TOR is TOR comprising 32 - 44.5 % abietic acid, 18 - 25 % dehydroabietic acid, 0 - 3 % dihydroabietic acid, 3.0 - 11.5 % isopimaric acid, 0 - 1.5 % 8,5-isopimaric acid, 0 - 2.5 % levopimaric acid, 3.3 - 4 % neobietic acid, 7.5 - 10 % palustric acid, 3 - 4.5 % pimaric acid and 0 - 4.0 % sandaropimaric acid. TOR may comprise < 0.1 % dimers and 0 - 7 % other components.

Any percentages described in this specification, unless otherwise indicated, may be understood as referring to % (w/w).

In an embodiment, the composition comprises wood rosin.

The term "wood rosin" may be understood as referring to a composition obtainable or obtained by distillation or other means from dead trees, tree stumps, branches etc. Wood rosin may comprise at least 10 % (w/w) of resin acids. Wood rosin may comprise 50 - 99 % (w/w) of resin acids, or typically (for example, in typical commercial preparations) 50 - 92% (w/w) of resin acids. An example of wood rosin comprises 45 - 51 % abietic acid, 7.9 - 8.5 % dehydroabietic acid, 0 - 1 % dihydroabietic acid, 11 - 15.5 % isopimaric acid, 0 - 4.2 % 8,5-isopimaric acid, 0 - 0.2 % levopimaric acid, 4.7 - 7 % neobietic acid, 8.2 - 10 % palustric acid, 3 - 7.1 % pimaric acid and 0 - 2.0 % sandaropimaric acid. Wood Rosin may comprise 0 - 4.2 % dimers and 0 - 1 % other components.

In an embodiment, the composition comprises gum rosin.

The term "gum rosin" may be understood as referring to a composition obtainable or obtained by distillation or separated by other means from resin harvested from a living tree. Gum rosin may comprise at least 10 % (w/w) of resin acids. Gum rosin may comprise 50 - 99 % (w/w) of resin acids, or typically (for example, in typical commercial preparations) 50 - 92 % (w/w) of resin acids. An example of gum rosin comprises 15 - 45 % abietic acid, 3 - 15 % dehydroabietic acid, 0 - 0.6 % dihydroabietic acid, 3.6 - 28 % isopimaric acid, 0 - 0.3 % 8,5-isopimaric acid, 0 - 1.8 % levopimaric acid, 10 - 19 % neobietic acid, 5 - 25 % palustric acid, 2 - 7.4 % pimaric acid and 0 - 1.5 % sandaropimaric acid. Gum rosin may comprise 0 - 1.0 % dimers and 0 - 3.5 % other components.

In an embodiment, the composition comprises DTO.

The term "distilled tall oil" or "DTO" may be understood as referring to a composition obtained by distillation of crude tall oil and further refinement of distilled tall oil. DTO may comprise at least 10 % (w/w) of resin acids. DTO typically comprises 10 - 60 % (w/w) of resin acids. In an embodiment, the DTO is a distillation fraction of tall oil. In an embodiment, the DTO is a mixture of DTO and a distillation fraction of tall oil. The distillation fraction of tall oil may be understood as referring to any resin acids containing fraction of CTO available or obtainable during CTO refining.

Apart from its main chemical constituents (cellulose, hemicellulose and lignin), wood also contains a wide variety of low molecular mass compounds known as wood extractives. Wood extractives may be separated from insoluble wood constituents e.g. by simple solvent extraction using polar or non-polar solvents.

Oleoresins may be obtained from coniferous trees or barks. Oleoresins may comprise resin and oil, for example an essential or fatty oil. Oleoresins may be obtainable by evaporation of the solvent(s) used for their production.

The composition may thus comprise e.g. at least one of the following: tall oil rosin (TOR), wood rosin, gum rosin, distilled tall oil (DTO), wood extractives, oleoresins, or any mixture or combination thereof.

In the context of this specification, the term "resin acids" may be understood as referring to various acidic compounds and/or a complex mixture of various acidic compounds derived from wood, in particular pine wood.

The exact composition of the one or more resin acids present in the resin acid based composition may vary e.g. according to the species of the trees from which the composition is obtained and the processing conditions under which it is manufactured. Resin acids may typically include compounds such as abietic acid, dehydroabietic acid, dehydroabietic acid, levopimaric acid, neoabietic acid, pimaric acid and isopimaric acid, only to mention a few. Other resin acids may be contemplated.

The resin acid based composition may comprise e.g. at least one of the following resin acids: abietic acid, dehydroabietic acid, levopimaric acid, neoabietic acid, pimaric acid, palustric acid, isopimaric acid, or any mixture or combination thereof.

In an embodiment, the resin acid based composition comprises at least one of following: abietic acid, dehydroabietic acid, palustric acid, neoabietic acid, pimaric acid, or isopimaric acid. The one or more resin acids (or a portion thereof) may, in general, be insoluble or poorly soluble in water.

The one or more resin acids may be saponified and/or dried. All of these forms may be effective against the dormant stage forms. Different forms may be more suitable to certain compositions and/or formulations depending e.g. on the composition; for example, in ethanol-based compositions, unmodified resin acids may be well suited, while more water-soluble forms of the resin acids may, at least in some embodiments, be better suited for water-based compositions.

In an embodiment, the one or more resin acids comprise or are resin acid(s) modified by saponification. Examples of a composition comprising saponified resin acids is rosin soap and compositions comprising rosin soap.

Various processes for the saponification of resin acids using e.g. NaOH or CaOH are known to a person skilled in the art.

In an embodiment, the composition comprises 1 - 90 % (w/w) of fatty acids and/or their derivatives. The fatty acids may be in form of oils or fats or in other forms, such as free fatty acids or esters, ethers, alkali metal salts, or fatty alcohols.

The composition and/or the one or more resin acids may include unsaponifiables, which do not have an acid group, for example, lipophilic neutral substances and/or esters from wood. The composition may include e.g. less than 15 % of unsaponifiables. The amount of unsaponifiables may typically be less than 5 % in DTO products and less than 6 % in TOR, wood and gum rosin.

In an embodiment, the one or more resin acids and/or the composition comprise or are resin acid(s) or a composition which is dried. They may be dried by spray drying, drum drying or by any other known suitable drying method. At least in some embodiments, the "dried" composition may have a moisture content of up to 15 % (w/w), or up to 10 % (w/w).

The composition may comprise e.g. about 0.1 - 50 % (w/w), or about 0.5 - 50 % (w/w) of the one or more resin acids, or about 0.1 - 10 % (w/w), or e.g. about 0.5 - 5 % (w/w), or about 1 - 5 % (w/w) of the one or more resin acids. As a skilled person will understand, the proportion of the one or more resin acids is not necessarily the same as the proportion of the rosin/resin product included in the composition, as the resin acids content of rosin/resin products may vary.

The composition may be, for example, a hand disinfectant, sanitizer, cleansing agent, cleaner, purifying agent, or detergent and/or a surface disinfectant or sanitizer.

The composition may comprise a solvent. The solvent may be selected e.g. such that the one or more resin acids and/or the resin/rosin product included therein is well soluble. Suitable solvents may include e.g. water (H₂O), an alcohol, such as ethanol, and/or any mixtures or combinations thereof. In embodiments in which the solvent is a mixture of water and an alcohol, such as ethanol, the ratio of water to the alcohol may be e.g. up to 70:30, or up to 50:50. Such a ratio may be suitable e.g. for compositions comprising one or more resin acids that are relatively water-soluble, such as saponified resin acids. Other possible solvents may include e.g. other alcohols, hydrocarbons and/or hydrocarbon-based solvents, vegetable oils and/or other oils, etc.

The composition may further comprise one or more additives, co-solvents, gelling agents, thickening agents, carriers, emulgators, etc. For example, additional co-solvents, such as ethanol, polypropylene glycol, 2-pyrrolidone, N-methyl pyrrolidone, N-ethyl pyrrolidone, and/or glycerol formal, may be included in the composition to improve its efficacy.

The composition may further comprise e.g. a compound or component for increasing penetration of resin acids across the coat of the dormant stage forms (such as a spore coat), a compound or component for promoting the germination of the dormant stage forms (such as the germination of spores), and/or a compound or component for enhancing adsorption / adherence of the one or more resin acids to the surface of the dormant stage forms (such as spore surface).

The composition may further comprise glutaraldehyde. Glutaraldehyde may assist in and/or enhance the sporicidal activity of the composition. In other words, compositions comprising glutaraldehyde may be particularly useful in killing and/or reducing the viability of dormant stage forms of a bacterium capable of forming the dormant stage forms.

The composition may comprise about 0.1 - 5 % (w/w), or 0.2 - 1.0 % (w/w) glutaraldehyde.

Other components, additives etc. may be contemplated.

### EXAMPLES

### EXAMPLE 1

Various compositions were tested for their ability to kill, reduce the viability of and/or prevent the germination of certain bacterial strains. The following products were made up in a 10% solution in their respective solvents:
Purified rosin (purified tall oil resin acid; prepared by purifying tall rosin; tall fatty acids <1-w%, tall resin acids >99-w%; sold under the trade name For-100 ULTRA) - absolute ethanol
Indonesian Gum Rosin (commercial grade of gum rosin originating from Indonesia; fatty acids <1-w%, resin acids >90-w%) - 10-w% absolute ethanol
Finnish tall oil rosin (resin acids >90-w%) - 10-w% absolute ethanol
Rosin Soap (sodium salts of finnish tall oil rosin, 10-w%; prepared by drying NaOH saponified tall rosin) - H₂O
Rosin-fatty acid (DTO) soap - Liquid Rosin soap (potassium salts of Finnish tall oil rosin and fatty acids, i.e. saponified DTO including also saponified fatty acids; potassium salts of Finnish tall oil rosin 10-w%) - H₂O

Properties of the test products are described also in Table 1.

**Table 1.**

| Test products w/w-% | | | | | | | |
|---|---|---|---|---|---|---|---|
| Active agent | Carboxylic formula | Rosins *1 | Fatty acids *1 | Others *2 | Water | Alcohol | Solubility |
| Rosin soap (solid) | -COONa | 91-94 | 3-6 | <4 | 0 | 0 | Water |
| Purified rosin | -COOH | 98-99 | 1 | <1 | 0 | 0 | Solvent |
| Gum Rosin | -COOH | 90, 1 | 0,4 | <10 | 0 | 0 | Solvent |
| Tall Rosin | -COOH | 92,2 | <5 | <4 | 0 | 0 | Solvent |
| Rosin-fatty acid (DTO) soap | -COOK | 23-25 | 39-40 | <4 | 11 | 24 (EtOH) | Water |

| | | | | | | | |
|---|---|---|---|---|---|---|---|
| *1 Rosin and fatty acid appeared in carboxylic formula *2 Neutral matter, other compounds or unknowns | | | | | | | |

*Clostridium difficile* strains were obtained from the Kel-vin Laboratories at the Royal Victoria Hospital, Belfast. The strains used were *C. difficile* TL178 (Clade 1, Ribotype 002), R20291 (Clade 2, Ribotype 027), CD305 (Clade 3, Ribotype 023), CF5 (Clade 4, Ribotype 017) and M120 (Clade 5, Ribotype 078). *C. difficile* cultures were incubated at 37°C for 48 hours in a Whitley A35 anaerobic workstation on pre-reduced fastidious anaerobe agar (FAABL: Oxoid, UK). ESKAPE pathogens (*Enterococcus faecium* DSM 25390, *Methicillin-Resistant Staphylococcus aureus* ATCC 33593, *Klebsiella pneumoniae* NCTC 204, *Acinetobacter baumannii* NCTC *13304, Pseudomonas aeruginosa* PA01 and *Enterobacter cloacae* NCTC 5920 were obtained from School of Pharmacy, Queen's University Belfast. The ESKAPE pathogens were incubated under aerobic conditions in a Binder BF046 cabinet (Binder, UK) on Mueller-Hinton agar (Oxoid, UK) at 37°C. *Candida albicans* NCYC 610 strain was obtained from School of Pharmacy, Queen's University Belfast.

*C. difficile* spore suspensions were prepared according to the method described by Wheeldon et al., (2008), British Journal of Nursing, 17(5), 316-320. Individual FAABL agar plates were inoculated with cells of TL178, R20291, CD305, CF5 and M120, and incubated anaerobically (N₂ /CO₂ /H₂) at 37°C for 72 h in the anaerobic chamber. Plates were subsequently incubated under aerobic conditions for 24hrs at 37°C following which colonies were harvested into 5 ml sterile PBS (Sigma Aldrich, UK) and shocked with absolute ethanol (50% [v/v]) for 1 h to kill vegetative cells, in accordance with the spore culture method of UK Standards for Microbiology Investigations (Wheeldon et al., 2008; UK Standards, 2014). The alcohol shocked suspensions were centrifuged at 3,000xg for 4 min and washed once in 5 ml sterile PBS with the resultant pellet resuspended in 70% (v/v) ethanol. Spore concentration was determined as approximately 1.3x10⁷ spores/ml, by viable spore counts (UK Standards for Microbiology Investigations, 2014).

### Assessment of sporicidal activity

To determine the effect of the resin acid containing products on spores of *C. difficile,* 100 µl of spores (ca. 1300 CFU) were suspended in 100 µl of substance at a final concentration of either 1%, 2.5% or 5% w/v or v/v active ingredient. Suspensions were incubated for 0 and 8 mins after which time treated spores were serially diluted in deionized water and cultured on Brazier's medium (Fannin, Ireland) anaerobically at 37°C for 48 h. Viable organisms were enumerated via Total Viable Counts of visible colonies on Brazier's agar after incubation. All testing was carried out in triplicate, with no treatment controls used as a comparison.

### Effect of resin acids against the ESKAPE pathogens and Candida albicans (not according to the present invention)

To assess the effect of Tall Oil based products on the ESKAPE pathogens, Rosin Soap was selected for testing. In contrast to the other products supplied, which are only soluble in ethanol, Rosin Soap is water soluble. This thus facilitates testing of the active ingredient against the ESKAPE pathogens as these isolates are known to be killed by ethanol. The effect of Rosin Soap on the ESKAPE pathogens was assayed according to British Standard BS EN 13704-2018. Cells of *E*. *faecium, S. aureus, K. pneumoniae, A. baumannii, P. aeruginosa* and *E. cloacae* were individually grown on Mueller-Hinton agar (MHA) plates overnight at 37°C from a bacterial store kept at -80°C. *Candida albicans* was grown in Sabouraud dextrose broth at 37°C for 48 hours from a bacterial store kept at - 80°C.

Bacterial colonies from inoculated plates were added to 5ml of quarter strength Ringers Solution (QSRS) and vortexed for 3 minutes. 5ml of *Candida albicans* was centrifuged at 2500rpm for 5 mins. The supernatant was poured off and the pellet was suspended in QSRS, this process was carried out twice. The suspension was diluted to an optical density (od) of 0.3 at 550nm (an od of 0.3 will contain approximately 10⁸ cfu/ml of bacteria). The suspension was diluted 1 in 50 in QSRS to achieve a bacterial cell concentration of approximately 10⁶ cfu/ml (if environmental conditions are being tested, 0.6% Bovine Serum Albumin should be added to the QSRS). Into empty wells add 100µl of bacterial suspension to 100µl of Rosin soap, mix well by withdrawing and expelling with the pipette 5 times. Start the timer. At time points of 30 seconds, 1 minute, 2 minutes, 3 minutes, 4 minutes and 5 minutes withdraw 20µl from the test wells and complete 10 fold dilutions to 10⁻⁴. Spot 20µl from each well onto MHA plates and incubate for 18 hours at 37°C. Record the number of colonies at each time point to determine the cfu/ml. Each of the ESKAPE pathogens and *Candida albicans* tested in triplicate against Rosin Soap, with triplicate technical replicates carried out for the determination of cfu/ml.

Time zero control was done by adding 100µl of bacterial suspension to 100µl of QSRS, mixing well by withdrawing and expelling with the pipette 5 times. Serially diluted to 10⁻⁴ as described above. Spotted 20µl from each well onto MHA plates and incubated for 18 hours at 37°C. Recorded the number of colonies at each time point to determine the cfu/ml. All assays were carried out in triplicate as described above.

A neutraliser control was required to see if the neutraliser has any effect on the treatment. Added 100µl of the test substance into a 96 well plate. Added 100µl of neutraliser buffer to the same well, mixed well by withdrawing and expelling with the pipette 5 times. Withdrew 100µl into a second well containing 100µl of 10⁶ bacterial suspension. Started the timer. After 5 minutes withdrew 20µl and serially diluted to 10⁻⁴ as outlined above. Spotted 20µl onto MHA plates and incubate for 18 hours at 37°C. All assays were carried out in triplicate as described above.

### EXAMPLE 2

The following compositions were used:
Rosin-fatty acid (DTO) soap - Liquid Rosin Soap (Potassium Salts of Finnish Tall Oil Rosin and Fatty Acids; available as a commercial composition under the trade name Rosetax by Forchem Oy) - Potassium salts of Finnish tall oil rosin 10-w% - H₂O
Rosin Soap (solid) - sodium salts of Finnish tall oil rosin
Purified rosin (solid) - purified Finnish tall oil rosin, >99 w-% resin acids, sold under the trade name For-100 ULTRA
Finnish tall oil rosin (solid) - commercial grade of tall rosin originated from Finland; 92 w-% resin acids
Indonesian gum rosin (solid) - gum resin acids > 90 w-%

Formulation of rosin acid samples involved dissolution of the rosin acids with absolute ethanol (HPLC grade, Sigma), at a concentration of 25% w/v. These samples were diluted with deionised water to reduce the concentration of ethanol to 70% v/v, 60% v/v, and 50% v/v respectively. If required, a surfactant, Tween^{®}80, SDS, or HEC were added at a final concentration of 3% w/v. Working solutions of each rosin acid sample were further diluted and tested at a final concentrations of 5% w/v.

### Efficacy of natural substances against five clades of Clostridium difficile

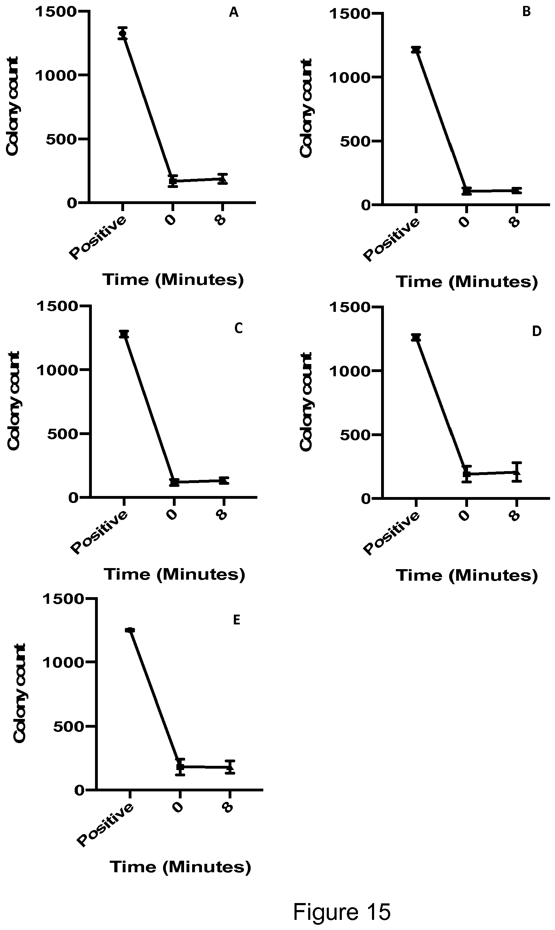

At 5% (w/v) (Finnish Tall Rosin, purified rosin, Indonesian Gum Rosin and Rosin Soap) and 5% (v/v) (rosin-fatty acid (DTO) soap) the five substances had the greatest efficacy against each of the five clades of *C. difficile* when compared to 2.5% and 1% (Figures 15- 29). The efficacy of each treatment at 5% (w/v) against each clade of *C. difficile* was >99% at 0 and 8 minutes. Substance efficacy against *C. difficile* at 2.5% (w/v; v/v) dropped to an average of 97.33% and 97.38% after 0 and 8 minutes respectively. At 1% (w/v; v/v) efficacy reduces to 84.72% and 86.35% after 0 and 8 minutes respectively. Using this method the exposure time did not affect the efficacy with similar efficacy seen at each time point. Spore controls and treatment only controls were carried out.

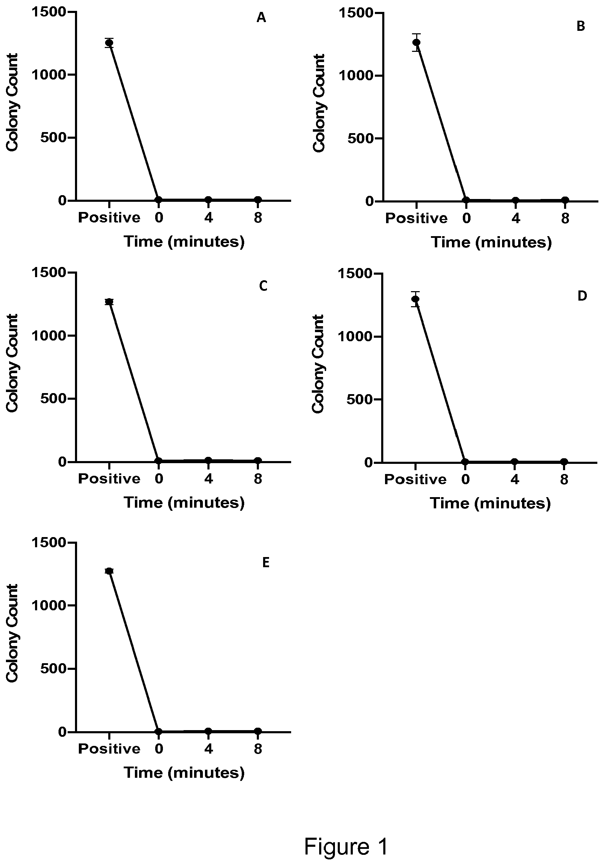

Figures 1A to 1E show the effect of Rosin soap (5 % w/v) against five clades of *Clostridium difficile,* A) R20291, B) M120, C) CF5, D) CD305, E) TL178 at 0, 4 and 8 minutes.

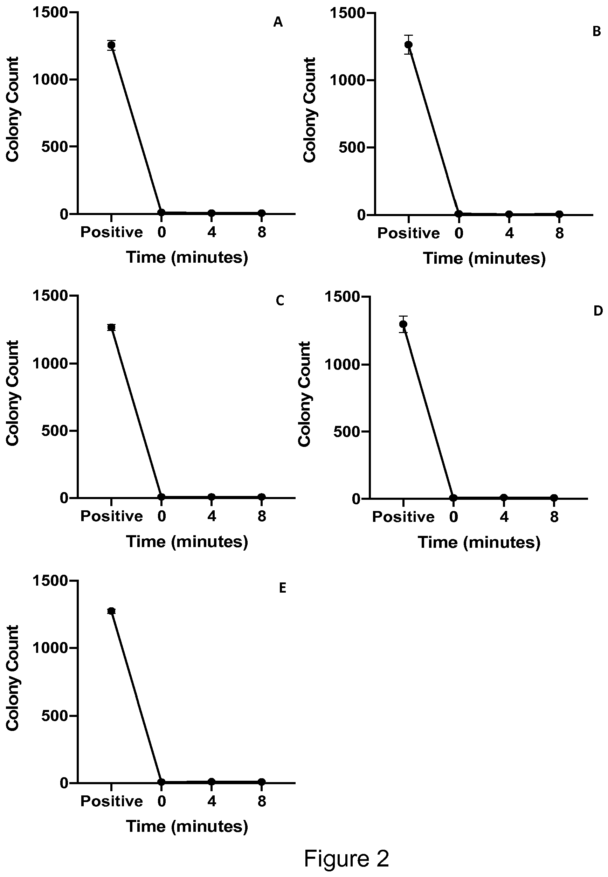

Figures 2A to 2E illustrate the effect of rosin-fatty acid (DTO) soap (5 % v/v) against five clades of *Clostridium difficile,* A) R20291, B) M120, C) CF5, D) CD305, E) TL178 at 0, 4 and 8 minutes.

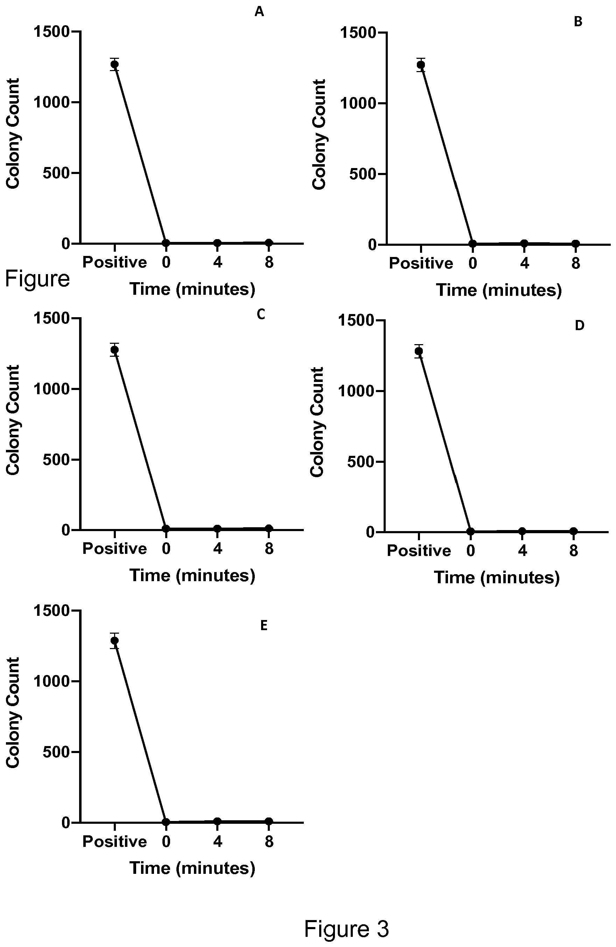

Figures 3A to 3E show the effect of Finnish Tall Rosin (5 % w/v) against five clades of *Clostridium difficile,* A) R20291, B) M120, C) CF5, D) CD305, E) TL178 at 0, 4 and 8 minutes.

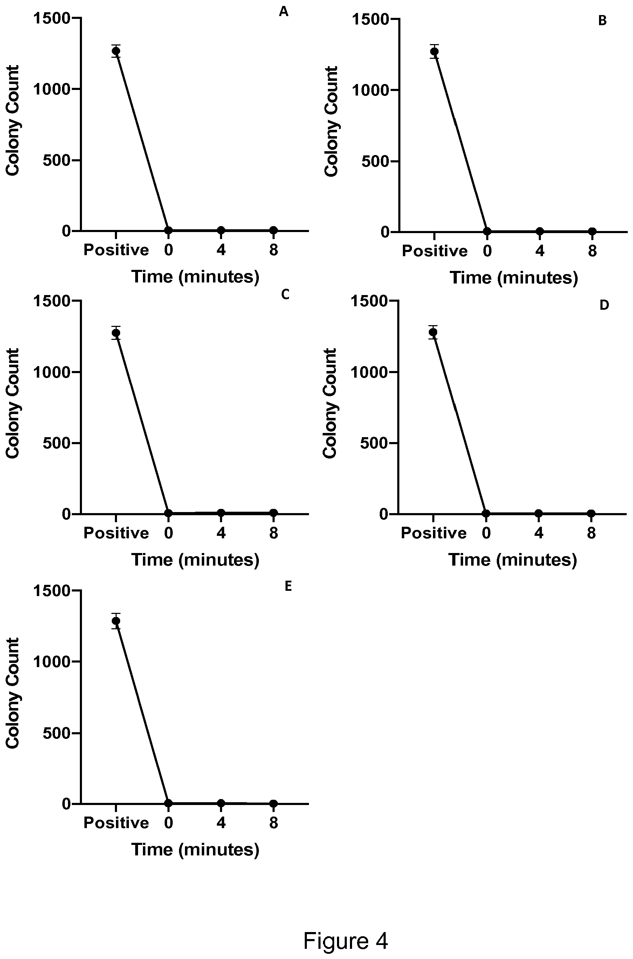

Figures 4A to 4E show the effect of Indonesian Gum Rosin (5 % w/v) against five clades of *Clostridium difficile,* A) R20291, B) M120, C) CF5, D) CD305, E) TL178 at 0, 4 and 8 minutes.

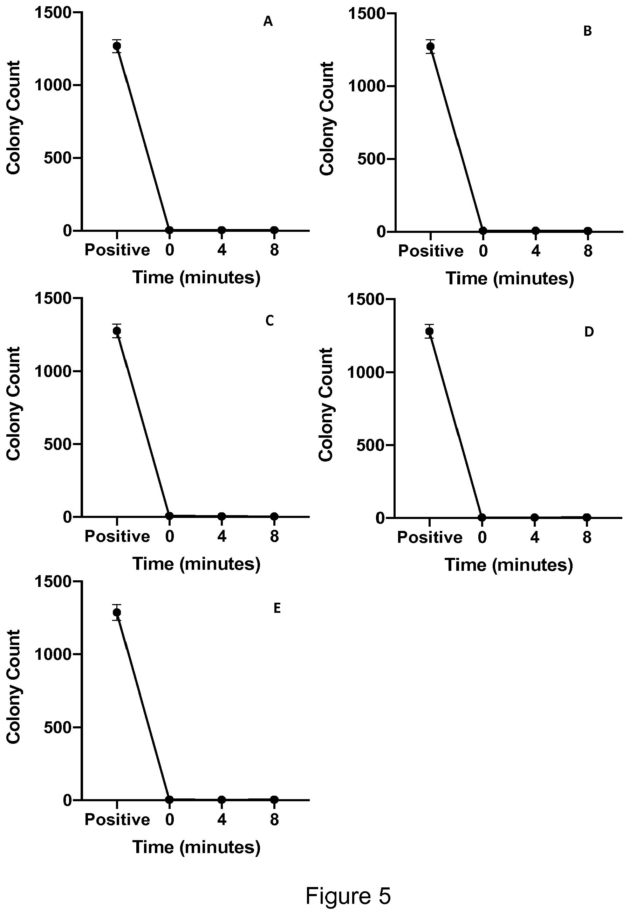

Figures 5A to 5E illustrate the effect of purified rosin (5 % w/v) against five clades of *Clostridium difficile,* A) R20291, B) M120, C) CF5, D) CD305, E) TL178 at 0, 4 and 8 minutes.

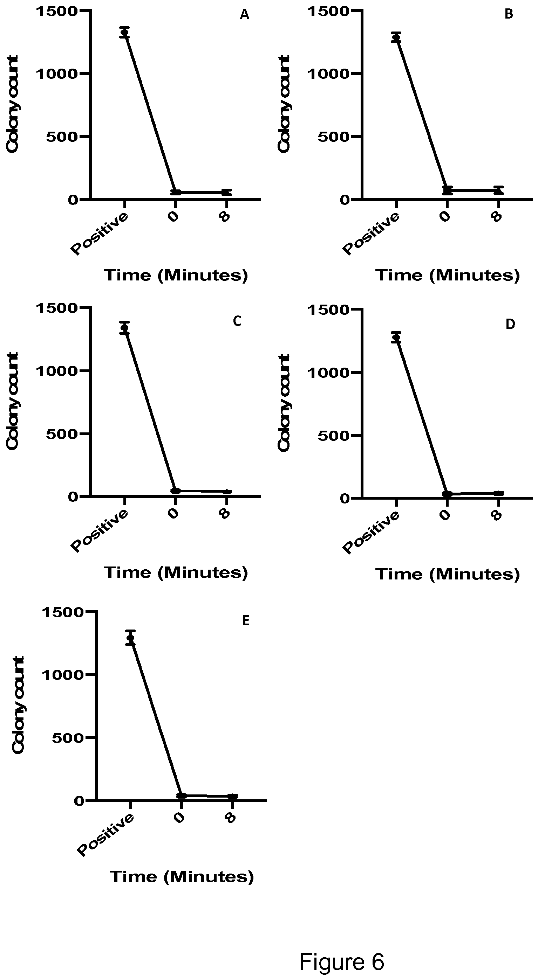

Figures 6A to 6E show the effect of Rosin Soap (2.5 % w/v) against five clades of *Clostridium difficile,* A) R20291, B) M120, C) CF5, D) CD305, E) TL178 at 0 and 8 minutes.

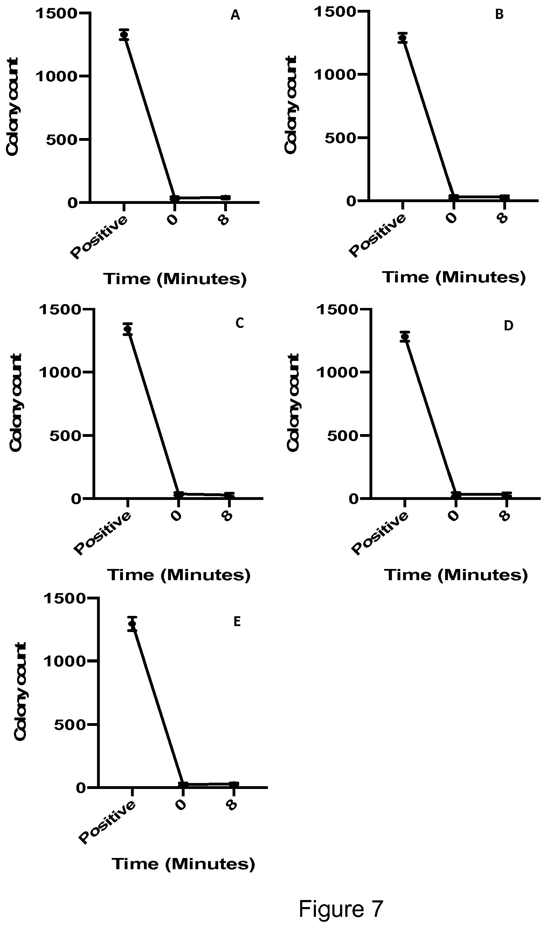

Figures 7A to 7E show the effect of rosin-fatty acid (DTO) soap (2.5 % v/v) against five clades of *Clostridium difficile,* A) R20291, B) M120, C) CF5, D) CD305, E) TL178 at 0 and 8 minutes.

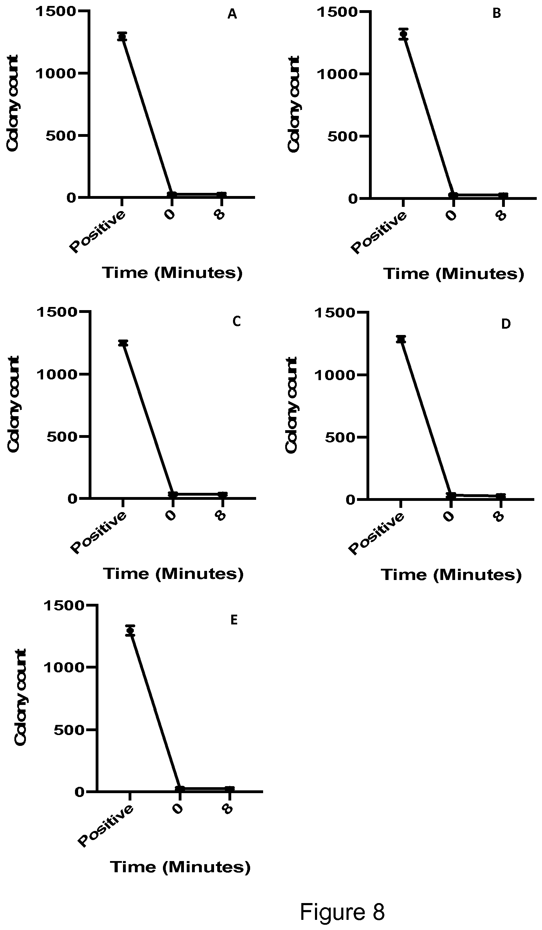

Figures 8A to 8E show the effect of Finnish Tall Oil (2.5 % w/v) against five clades of *Clostridium difficile,* A) R20291, B) M120, C) CF5, D) CD305, E) TL178 at 0 and 8 minutes.

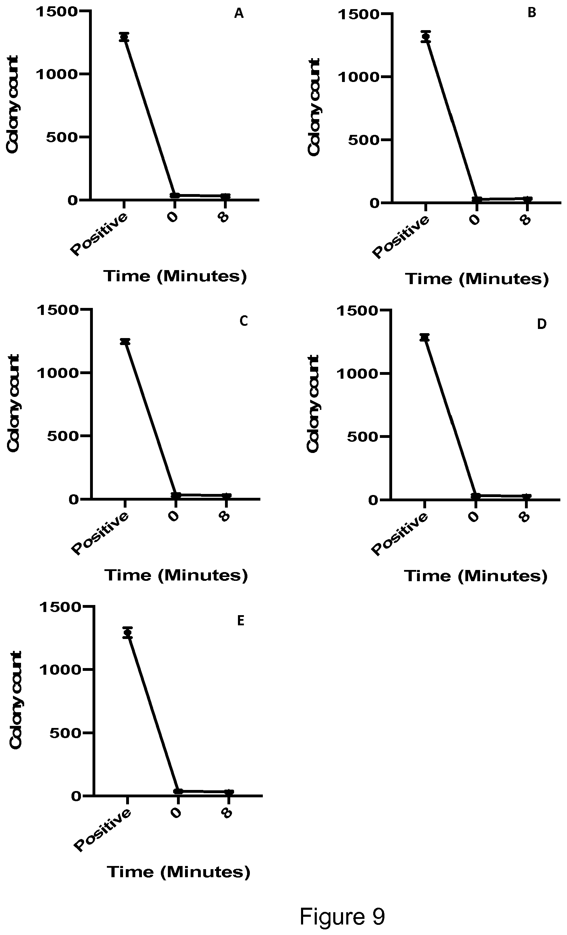

Figures 9A to 9E show the effect of Indonesian Gum Rosin (2.5 % w/v) against five clades of *Clostridium difficile,* A) R20291, B) M120, C) CF5, D) CD305, E) TL178 at 0 and 8 minutes.

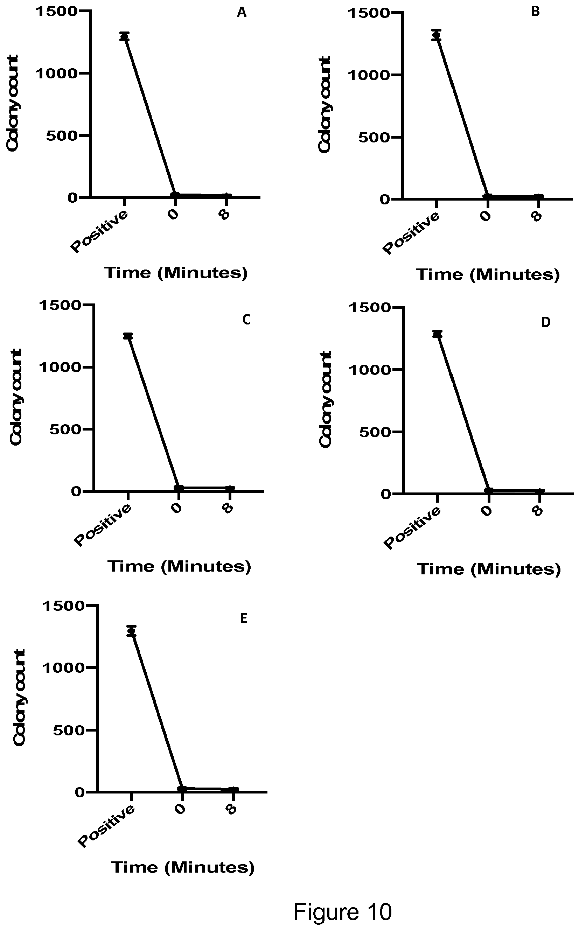

Figures 10A to 10E show the effect of purified rosin (2.5 % w/v) against five clades of *Clostridium difficile,* A) R20291, B) M120, C) CF5, D) CD305, E) TL178 at 0 and 8 minutes.

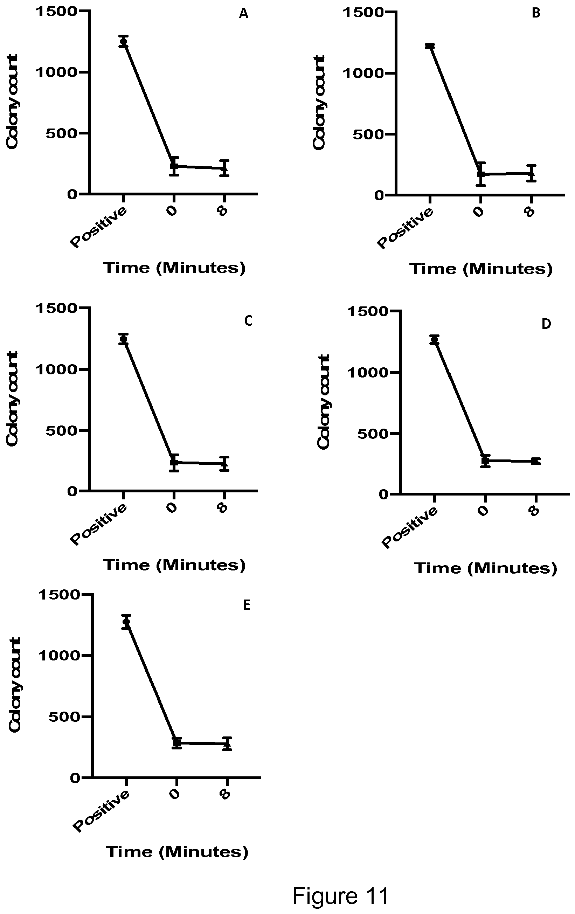

Figures 11A to 11E show the effect of Rosin Soap (1 % w/v) against five clades of *Clostridium difficile,* A) R20291, B) M120, C) CF5, D) CD305, E) TL178 at 0 and 8 minutes.

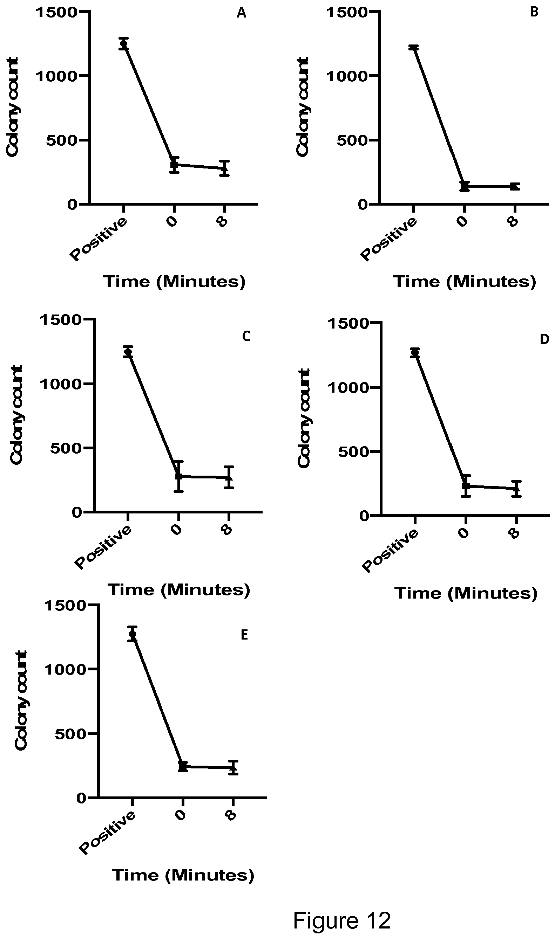

Figures 12A to 12E show the effect of rosin-fatty acid (DTO) soap (1 % v/v) against five clades of *Clostridium difficile,* A) R20291, B) M120, C) CF5, D) CD305, E) TL178 at 0 and 8 minutes.

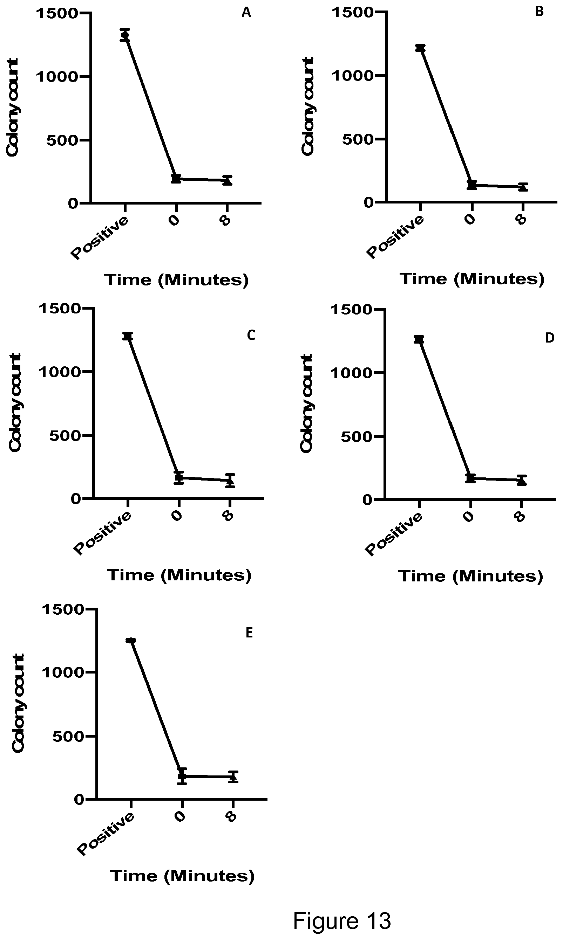

Figures 13A to 13E show the effect of Finnish Tall Oil (1 % w/v) against five clades of *Clostridium difficile,* A) R20291, B) M120, C) CF5, D) CD305, E) TL178 at 0 and 8 minutes.

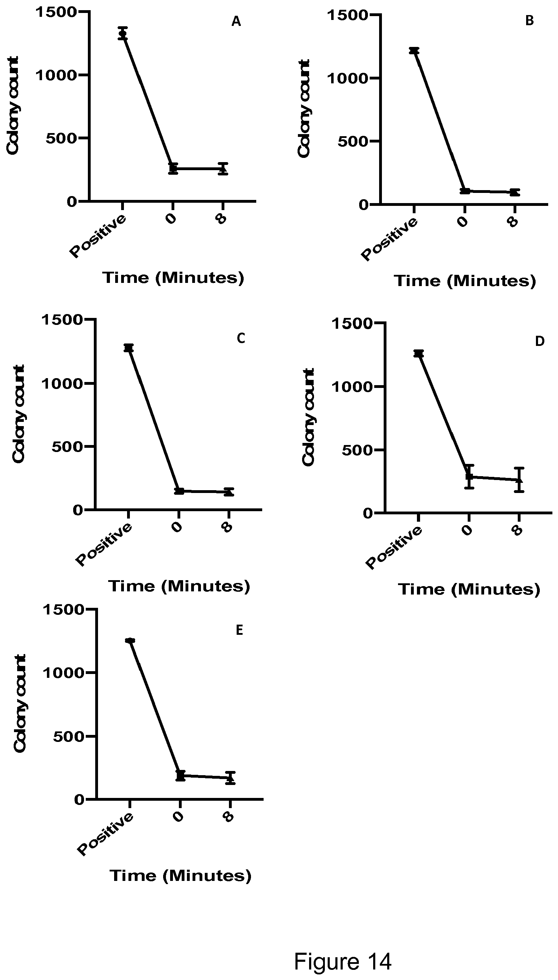

Figures 14A to 14E illustrate the effect of Indonesian Gum Rosin (1 % w/v) against five clades of *Clostridium difficile,* A) R20291, B) M120, C) CF5, D) CD305, E) TL178 at 0 and 8 minutes.

Figures 15A to 15E show the effect of purified rosin (1 % w/v) against five clades of *Clostridium difficile,* a) R20291, b) M120, c) CF5, d) CD305, e) TL178 at 0 and 8 minutes.

In all of the figures 1A to 15E, the results are the means of three biological and two technical triplicates. Positive denotes the initial number of spores used in the assay.

### EXAMPLE 3 - not according to the present invention

### Efficacy of Rosin soap against the ESKAPE pathogens in 'clean' conditions

Rosin Soap (sodium salts of Finnish tall oil rosin) (5% w/v) was tested against the six ESKAPE pathogens, five of which showed at least a 3-log reduction following 5 minute exposures (n=2; Figures 16-21). The greatest efficacy was observed against *A. baumannii* and *E. faecium* (Gram-positive and Gram-negative, respectively), with cessation of growth observed after 1 minute exposure. No further bacterial growth was seen after 2-minute exposures for both methicillin-resistant *S. aureus* and *P. aeruginosa. E. cloacae* was not killed within 5 minutes; however, a three-log reduction in colony numbers occurred between 0 and 5 minutes. *K*. *pneumoniae* was most resistant to treatment by Rosin soap; only a one-log reduction in colony numbers was observed between 0 and 5 minutes.

Fig. 16 shows the effect of Rosin soap (5% w/v) after 0, 0.5, 1, 2 ,3, 4 and 5mins exposure against the ESKAPE pathogen *Acinetobacter baumannii.* Fig. 17 shows the effect of Rosin soap (5% w/v) after 0, 0.5, 1, 2, 3, 4 and 5mins exposure against the ESKAPE pathogen *Enterococcus faecium.* Fig. 18 shows the effect of Rosin soap (5% w/v) after 0, 0.5, 1, 2, 3, 4 and 5mins exposure against the ESKAPE pathogen *Methicillin-* resistant *Staphylococcus aureus.*

Fig. 19 demonstrates the effect of Rosin soap (5% w/v) after 0, 0.5, 1, 2, 3, 4 and 5mins exposure against the ESKAPE pathogen *Pseudomonas aeruginosa.*

Fig. 20 shows the effect of Rosin soap (5% w/v) after 0, 0.5, 1, 2, 3, 4 and 5mins exposure against the ESKAPE pathogen *Enterobacter cloacae.*

Fig. 21 shows the effect of Rosin soap (5% w/v) after 0, 0.5, 1, 2, 3, 4 and 5mins exposure against the ESKAPE pathogen *Klebsiella pneumoniae.*

In all of Figs. 16 to 21, the results are the means of three biological and three technical triplicates.

### Efficacy of Rosin soap against the ESKAPE pathogens in 'dirty' conditions

For any disinfection study it is important to determine the effect of inferring substances on product efficacy. This involves including some form of organic contamination into the disinfection assay e.g. bovine serum albumin. To determine the effect of organic contamination on the antimicrobial activity of Rosin Soap, the assays described above were repeated in the presence of bovine serum albumin (6g/l): the British standard for organic contamination is 3g/l) bovine serum albumin. Rosin Soap (5% w/v) was tested against the six ESKAPE pathogens, all of which showed at least a 3-log reduction following 5 minute exposures (n=2; Figures 22-27) in the presence of bovine serum albumin 6g/l - 0.6%). The greatest efficacy was observed against *E. faecium* (Gram-negative), with cessation of growth noted after 1 minute exposure (Figure 22). After two minutes of exposure *A*. *baumannii, P. aeruginosa* and methicillin-resistant *S. aureus* no further bacterial growth was seen (Figures 23-25). *E. cloacae* and *K. pneumoniae* were not killed within 5 minutes; however, a three-log reduction in colony numbers occurred between 0 and 5 minutes (Figures 26-27). Results recorded in the presence of organic matter were similar to those recorded in the absence of organic matter. In 'clean' conditions *A*. *baumannii* ceased growing after 1 minute whilst in 'dirty' conditions it was 2 minutes.

Fig. 22 shows the effect of Rosin soap (5 % w/v) in the presence of 0.6% bovine serum albumin after 0, 0.5, 1, 2, 3, 4 and 5mins exposure against the ESKAPE pathogen *Enterococcus faecium.*

Fig. 23 illustrates the effect of Rosin soap (5 % w/v) in the presence of 0.6% bovine serum albumin after 0, 0.5, 1, 2, 3, 4 and 5mins exposure against the ESKAPE pathogen *Acinetobacter baumannii.*

Fig. 24 shows the effect of Rosin soap (5 % w/v) in the presence of 0.6% bovine serum albumin after 0, 0.5, 1, 2, 3, 4 and 5mins exposure against the ESKAPE pathogen *Pseudomonas aeruginosa.*

Fig. 25 demonstrates the effect of Rosin soap (5 % w/v) in the presence of 0.6% bovine serum albumin after 0, 0.5, 1, 2, 3, 4 and 5mins exposure against the ESKAPE pathogen *Methicillin-*resistant *Staphylococcus aureus.*

Fig. 26 shows the effect of Rosin soap (5 % w/v) in the presence of 0.6% bovine serum albumin after 0, 0.5, 1, 2, 3, 4 and 5mins exposure against the ESKAPE pathogen *Enterobacter cloacae.*

Fig. 27 shows the effect of Rosin soap (5 % w/v) in the presence of 0.6% bovine serum albumin after 0, 0.5, 1, 2, 3, 4 and 5mins exposure against the ESKAPE pathogen *Klebsiella pneumoniae.*

In all of Figs. 22 to 27, the results are the means of three biological and three technical triplicates.

### Efficacy of Rosin soap against Candida albicans 'clean' conditions

Rosin soap (5% w/v) had minimal efficacy on the yeast *Candida albicans.* After Rosin soap treatment of 5 minutes a one log reduction was seen after 5 minutes (Figure 28).

Figure 28 illustrates the effect of Rosin soap (5% w/v) after 0, 0.5, 1, 2, 3, 4 and 5mins exposure against *Candida albicans.* The results are the means of three biological and three technical triplicates.

### Efficacy of Rosin soap against Candida albicans 'dirty' conditions

To determine the effect of organic contamination on the antimicrobial activity of Rosin Soap, the assay described above was repeated in the presence of bovine serum albumin (6g/l): the British standard for organic contamination is 3g/l) bovine serum albumin. Rosin soap (5% w/v) had minimal efficacy on the yeast *Candida albicans.* Rosin soap treatment did not contribute to log reductions in yeast colonies after 5 minutes.

Figure 29 shows the effect of Rosin soap (5 % w/v) in the presence of 0.6% bovine serum albumin after 0, 0.5, 1, 2, 3, 4 and 5mins exposure against *Candida albicans.* The results are the means of three biological and three technical triplicates.

### EXAMPLE 4

Various formulations of the compositions were made as shown in Tables 2 to 5. The formulations are indicated such that e.g. the composition with 1 % w/w of Rosin soap in the first row of Table 1 contains 1 g of Rosin soap, 3 g of HEC and 96 g of solvent. Hydroxyethyl cellulose (HEC) is a gelling or thickening agent which was added to Rosin soap, Indonesian gum rosin, Finnish Tall Rosin and purified rosin. HEC concentrations of 2% to 5% seemed well suitable to obtain a spreadable gel (Tables 2-5). Precipitation of substance was observed when the H₂O to Ethanol ratio was 50:50 (Tables 3-5).

**Table 2. Solubility of Rosin soap**

| **Active agent** | **% of active agent*** | **Solvent (H₂0 : Ethanol)** | **% of Hydroxy-ethyl cellulose** | **Description** |
|---|---|---|---|---|
| **Rosin soap (solid)** | 1% w/w | 70:30 | 3% | Liquid-like gel |
| | 2.5% w/w | 70:30 | 3% | Liquid-like gel |
| | 5% w/w | 70:30 | 3% | Liquid-like gel |

| | | | | |
|---|---|---|---|---|
| *Proportion of rosin soap of the entire composition | | | | |

**Table 3. Solubility of Indonesian gum rosin**

| **Active agent** | **% of active agent*** | **Solvent (H₂0: Ethanol)** | **% of Hydroxyethyl cellulose** | **Description** |
|---|---|---|---|---|
| **Indonesian gum rosin (solid)**, **>90%** | 1% w/w | 70:30 | 3% | Liquid-like gel |
| | | 50:50 | 3% | Solid-like gel, resin precipitation observed |
| | 2.5% w/w | 70:30 | 4% | Liquid-like gel |
| | | 50:50 | 3% | Solid-like gel, resin precipitation observed |

| | | | | |
|---|---|---|---|---|
| *Proportion of Indonesian gum rosin of the entire composition | | | | |

**Table 4. Solubility of Finnish tall rosin**

| **Active agent** | **% of active agent*** | **Solvent (H₂0: Ethanol)** | **% of Hydroxy-ethyl cellulose** | **Description** |
|---|---|---|---|---|
| **Finnish tall rosin (solid)** | 1% w/w | 70:30 | 5% | Solid-like gel |
| | | 50:50 | 2% | Solid-like gel, resin precipitation observed |
| | 2.5% w/w | 70:30 | 5% | Solid-like gel |
| | | 50:50 | 1.50% | Liquid-like gel, resin precipitation observed |

| | | | | |
|---|---|---|---|---|
| *Proportion of Finnish tall oil rosin of the entire composition | | | | |

**Table 5: Solubility of For-100 ULTRA**

| **Active agent** | **% of active agent*** | **Solvent (H₂0: Ethanol)** | **% of Hydroxyethyl cellulose** | **Description** |
|---|---|---|---|---|
| **Purified rosin (solid)** | 1% w/w | 70:30 | 5% | Liquid-like gel |
| | | 50:50 | 3% | Solid-like gel, resin precipitation observed |
| | 2.5% w/w | 70:30 | 5% | Liquid-like gel |
| | | 50:50 | 2% | Solid-like gel, resin precipitation observed |

### EXAMPLE 5

### Addition of chemical disruptors/germinants to treatments to aid sporicidal efficacy

Into empty wells add 100µl of spore suspension to 100µl of Rosin soap or Rosetax-21 and 50 µl of chemical enhancer/adjuvant, mix well by withdrawing and expelling with the pipette 5 times. Start the timer. At time points of 30 seconds, 1 minute, 2 minutes, 3 minutes, 4 minutes, and 5 minutes withdraw 20µl from the test wells and complete 10-fold dilutions to 10⁻⁴ in Dey Engley neutralising broth. Spot 20µl from each well onto Brazier's agar plates and incubate for 48 hours at 37°C in anaerobic conditions. Record the number of colonies at each time point to determine the cfu/ml. A variety of chemical disruptors/ germinants were examined (Tables 6-8).

**Table 6: Chemical disruptor solutions used.**

| **CHEMICAL DISRUPTOR** | **Concentration** | **CONTACT TIME** |
|---|---|---|
| **Glutaraldehyde** | 0.5 % | 10 min |
| **Sodium dichloroisocyanurate (NaDCC)** | 500 ppm | 10 min |
| **Hydrogen peroxide (H₂O₂)** | 4% | 15 min, 6hrs, 24hrs |
| **N-methylpyrrolidine** | 5% | 10 min, 1hr |
| **2-pyrrolidone** | 5% | 10 min, 1hr |
| **Dimethylacetamide** | 5% | 10 min, 1hr |
| **Glycerol Formal** | 5% | 10 min, 1hr |

**Table 7: The composition of germinant solutions used.**

| | **GERMINANT SOLUTION COMPONENTS** | | | | |
|---|---|---|---|---|---|
| **Germinant solution** | **Sodium taurocholate** | **Calcium chloride** | **Glycine** | **L-cystine** | **Thioglycolic acid** |
| 1 | + | + | - | - | - |
| 2 | + | - | + | - | - |
| 3 | + | + | + | - | - |
| 4 | + | + | + | + | + |

**Table 8: The concentrations of individual germinant solution components used.**

| **GERMINANT SOLUTION COMPONENT** | **Concentration** |
|---|---|
| **Sodium taurocholate** | 2.5 % |
| **Calcium chloride** | 150 mM |
| **Glycine** | 100 mM |
| **L-cystine** | 0.125 % |
| **Thioglycolic acid** | 0.125 % |

The efficacy of Rosetax-21 (2.5%w/v) and Rosin Soap Powder (2.5%w/v) in the presence of a chemical disruptors/ germinants was aided by one that was examined. Rosetax-21 and Rosin Soap Powder when in the presence 0.5% glutaraldehyde had 100% efficacy against two strains of *Clostridium difficile* (Figures 30 to 33). All Glutaraldehyde experiments were carried out with a Glutaraldehyde, H₂O and treatment only control. Glutaraldehyde was the only chemical disruptor/germinant that contributed to sporicidal activity of Rosin based treatments (Rosin soap powder and Rosetax-21). The other chemical disruptors/germinants did not aid the efficacy of Rosetax-21 (5%) or Rosin soap powder (data not shown).

## Claims

1. A non-therapeutic method for killing, reducing the viability of, and/or preventing the germination of spores of a spore-forming bacterium, wherein the method comprises applying a composition comprising one or more resin acids to the spores of the bacterium, thereby killing, reducing the viability of, and/or preventing the germination of the spores of the bacterium at least partially.

2. The method according to claim 1, wherein the composition comprising the one or more resin acids comprises, is, or consists of at least one of tall oil rosin (TOR), wood rosin, gum rosin, distilled tall oil (DTO), wood extractives, oleoresins, or any mixture or combination thereof; and/or wherein the composition comprises at least one of the following resin acids: abietic acid, dehydroabietic acid, levopimaric acid, neoabietic acid, pimaric acid, palustric acid, or isopimaric acid.

3. The method according to claim 1 or 2, wherein the one or more resin acids are saponified and/or dried.

4. The method according to any one of claims 1 - 3, wherein the composition is a hand disinfectant, sanitizer, cleansing agent, cleaner, purifying agent, or detergent and/or a surface disinfectant or sanitizer.

5. The method according to any one of claims 1 - 4, wherein the bacterium is a Gram-positive bacterium; wherein the bacterium is optionally a bacterium of the genus *Bacillus* and/or *Clostridium,* such as a bacterium of the species *Clostridium difficile* or *Clostridium perfringens.*

6. The method according to any one of claims 1 - 5, wherein the composition comprises about 0.1 - 50 % (w/w), or about 0.1 - 10 % (w/w), or about 0.5 - 5 % (w/w) of the one or more resin acids.

7. The method according to any one of claims 1 - 6, wherein the composition further comprises glutaraldehyde, wherein the composition optionally comprises about 0.1 - 5 % (w/w) glutaraldehyde.

8. Non-therapeutic use of a composition comprising one or more resin acids for killing, reducing the viability of, and/or preventing the germination of spores of a spore-forming bacterium, wherein the composition is applied to the spores of the bacterium.

9. The use according to claim 8, wherein the composition comprising the one or more resin acids comprises, is, or consists of at least one of tall oil rosin (TOR), wood rosin, gum rosin, distilled tall oil (DTO), wood extractives, oleoresins, or any mixture or combination thereof; and/or wherein the composition comprises at least one of the following resin acids: abietic acid, dehydroabietic acid, levopimaric acid, neoabietic acid, pimaric acid, palustric acid, or isopimaric acid.

10. The use according to claim 8 or 9, wherein the one or more resin acids are saponified and/or dried.

11. The use according to any one of claims 8 - 10, wherein the composition is a hand disinfectant, sanitizer, cleansing agent, cleaner, purifying agent, or detergent and/or a surface disinfectant or sanitizer.

12. The use according to any one of claims 8 - 11, wherein the bacterium is a Gram-positive bacterium; wherein the bacterium is optionally a bacterium of the genus *Bacillus* and/or *Clostridium,* such as a bacterium of the species *Clostridium difficile* or *Clostridium perfringens.*

13. The use according to any one of claims 8 - 12, wherein the composition comprises about 0.1 - 50 % (w/w), or about 0.1 - 10 % (w/w), or about 0.5 - 5 % (w/w) of the one or more resin acids.

14. The use according to any one of claims 8 - 13, wherein the composition further comprises glutaraldehyde, wherein the composition optionally comprises about 0.1 - 5 % (w/w) glutaraldehyde.

## Patentansprüche

1. Nicht-therapeutisches Verfahren zum Abtöten, zur Verringerung der Lebensfähigkeit und/oder zur Verhinderung der Keimung von Sporen eines sporenbildenden Bakteriums, wobei das Verfahren das Aufbringen einer Zusammensetzung, die eine oder mehrere Harzsäuren umfasst, auf die Sporen des Bakteriums umfasst, wodurch die Sporen des Bakteriums zumindest teilweise abgetötet, in ihrer Lebensfähigkeit verringert und/oder an der Keimung gehindert werden.

2. Verfahren nach Anspruch 1, wobei die Zusammensetzung, die die eine oder die mehreren Harzsäuren umfasst, mindestens eines von Tallölharz (Tall Oil Rosin, TOR), Holzharz, Gummiharz, destilliertem Tallöl (DTO), Holzextraktstoffen, Oleoresinen oder einer beliebigen Mischung oder Kombination davon umfasst, ist oder daraus besteht; und/oder wobei die Zusammensetzung mindestens eine der folgenden Harzsäuren umfasst: Abietinsäure, Dehydroabietinsäure, Levopimarsäure, Neoabietinsäure, Pimarsäure, Palustrinsäure oder Isopimarsäure.

3. Verfahren nach Anspruch 1 oder 2, wobei die eine oder die mehreren Harzsäuren verseift und/oder getrocknet sind.

4. Verfahren nach einem der Ansprüche 1 - 3, wobei die Zusammensetzung ein Handdesinfektionsmittel, ein Hygienemittel, ein Reinigungsmittel, ein Reiniger, ein Säuberungsmittel oder ein Detergens und/oder ein Oberflächendesinfektionsmittel oder Oberflächenhygienemittel ist.

5. Verfahren nach einem der Ansprüche 1 - 4, wobei das Bakterium ein grampositives Bakterium ist; wobei das Bakterium optional ein Bakterium der Gattung *Bacillus* und/oder *Clostridium* ist, wie etwa ein Bakterium der Art *Clostridium difficile* oder *Clostridium perfringens.*

6. Verfahren nach einem der Ansprüche 1 - 5, wobei die Zusammensetzung etwa 0,1 - 50 Gew.-% oder etwa 0,1 - 10 Gew.-% oder etwa 0,5 - 5 Gew.-% der einen oder der mehreren Harzsäuren umfasst.

7. Verfahren nach einem der Ansprüche 1 - 6, wobei die Zusammensetzung weiterhin Glutaraldehyd umfasst, wobei die Zusammensetzung optional etwa 0,1 - 5 Gew.-% Glutaraldehyd umfasst.

8. Nicht-therapeutische Verwendung einer Zusammensetzung, die eine oder mehrere Harzsäuren umfasst, zum Abtöten, zur Verringerung der Lebensfähigkeit und/oder zur Verhinderung der Keimung von Sporen eines sporenbildenden Bakteriums, wobei die Zusammensetzung auf die Sporen des Bakteriums aufgebracht wird.

9. Verwendung nach Anspruch 8, wobei die Zusammensetzung, die die eine oder die mehreren Harzsäuren umfasst, mindestens eines von Tallölharz (Tall Oil Rosin, TOR), Holzharz, Gummiharz, destilliertem Tallöl (DTO), Holzextraktstoffen, Oleoresinen oder einer beliebigen Mischung oder Kombination davon umfasst, ist oder daraus besteht; und/oder wobei die Zusammensetzung mindestens eine der folgenden Harzsäuren umfasst: Abietinsäure, Dehydroabietinsäure, Levopimarsäure, Neoabietinsäure, Pimarsäure, Palustrinsäure oder Isopimarsäure.

10. Verwendung nach Anspruch 8 oder 9, wobei die eine oder die mehreren Harzsäuren verseift und/oder getrocknet sind.

11. Verwendung nach einem der Ansprüche 8 - 10, wobei die Zusammensetzung ein Handdesinfektionsmittel, ein Hygienemittel, ein Reinigungsmittel, ein Reiniger, ein Säuberungsmittel oder ein Detergens und/oder ein Oberflächendesinfektionsmittel oder Oberflächenhygienemittel ist.

12. Verwendung nach einem der Ansprüche 8 - 11, wobei das Bakterium ein grampositives Bakterium ist; wobei das Bakterium optional ein Bakterium der Gattung *Bacillus* und/oder *Clostridium* ist, wie etwa ein Bakterium der Art *Clostridium difficile* oder *Clostridium perfringens.*

13. Verwendung nach einem der Ansprüche 8 - 12, wobei die Zusammensetzung etwa 0,1 - 50 Gew.-% oder etwa 0,1 - 10 Gew.-% oder etwa 0,5 - 5 Gew.-% der einen oder der mehreren Harzsäuren umfasst.

14. Verwendung nach einem der Ansprüche 8 - 13, wobei die Zusammensetzung weiterhin Glutaraldehyd umfasst, wobei die Zusammensetzung optional etwa 0,1 - 5 Gew.- % Glutaraldehyd umfasst.

## Revendications

1. Procédé non thérapeutique pour tuer, réduire la viabilité et/ou empêcher la germination de spores d'une bactérie sporulée, dans lequel le procédé comprend l'application d'une composition comprenant un ou plusieurs acides résiniques aux spores de la bactérie, tuant ainsi, réduisant la viabilité et/ou empêchant la germination des spores de la bactérie au moins partiellement.

2. Procédé selon la revendication 1, dans lequel la composition comprenant lesdits un ou plusieurs acides résiniques comprend, est, ou consiste en au moins un de colophane d'huile de tall (TOR), colophane de bois, colophane de gomme, huile de tall distillée (DTO), extraits de bois, oléorésines, ou tout mélange ou combinaison de ceux-ci ; et/ou dans lequel la composition comprend au moins un des acides résiniques suivants : acide abiétique, acide déhydroabiétique, acide lévopimarique, acide néoabiétique, acide pimarique, acide palustrique ou acide isopimarique.

3. Procédé selon la revendication 1 ou la revendication 2, dans lequel lesdits un ou plusieurs acides résiniques sont saponifiés et/ou séchés.

4. Procédé selon l'une quelconque des revendications 1 à 3, dans lequel la composition est un désinfectant pour les mains, un assainissant, un agent nettoyant, un produit nettoyant, un agent purifiant, ou un détergent et/ou un désinfectant ou assainissant de surface.

5. Procédé selon l'une quelconque des revendications 1 à 4, dans lequel la bactérie est une bactérie à Gram positif ; dans lequel la bactérie est facultativement une bactérie du genre Bacillus et/ou Clostridium, telle qu'une bactérie de l'espèce Clostridium difficile ou Clostridium perfringens.

6. Procédé selon l'une quelconque des revendications 1 à 5, dans lequel la composition comprend environ 0,1 à 50 % en poids/poids (p/p), ou environ 0,1 à 10 % en poids/poids (p/p), ou environ 0,5 à 5 % en poids/poids (p/p) desdits un ou plusieurs acides résiniques.

7. Procédé selon l'une quelconque des revendications 1 à 6, dans lequel la composition comprend en outre du glutaraldéhyde, dans lequel la composition comprend facultativement environ 0,1 à 5 % en poids/poids (p/p) de glutaraldéhyde.

8. Utilisation non thérapeutique d'une composition comprenant un ou plusieurs acides résiniques pour tuer, réduire la viabilité et/ou empêcher la germination de spores d'une bactérie sporulée, dans laquelle la composition est appliquée aux spores de la bactérie.

9. Utilisation selon la revendication 8, dans laquelle la composition comprenant lesdits un ou plusieurs acides résiniques comprend, est, ou consiste en au moins un de colophane d'huile de tall (TOR), colophane de bois, colophane de gomme, huile de tall distillée (DTO), extraits de bois, oléorésines, ou tout mélange ou combinaison de ceux-ci ; et/ou dans laquelle la composition comprend au moins un des acides résiniques suivants : acide abiétique, acide déhydroabiétique, acide lévopimarique, acide néoabiétique, acide pimarique, acide palustrique ou acide isopimarique.

10. Utilisation selon la revendication 8 ou la revendication 9, dans laquelle lesdits un ou plusieurs acides résiniques sont saponifiés et/ou séchés.

11. Utilisation selon l'une quelconque des revendications 8 à 10, dans laquelle la composition est un désinfectant pour les mains, un assainissant, un agent nettoyant, un produit nettoyant, un agent purifiant, ou un détergent et/ou un désinfectant ou assainissant de surface.

12. Utilisation selon l'une quelconque des revendications 8 à 11, dans laquelle la bactérie est une bactérie à Gram positif ; dans laquelle la bactérie est facultativement une bactérie du genre *Bacillus* et/ou *Clostridium,* telle qu'une bactérie de l'espèce *Clostridium difficile* ou *Clostridium perfringens.*

13. Utilisation selon l'une quelconque des revendications 8 à 12, dans laquelle la composition comprend environ 0,1 à 50 % en poids/poids (p/p), ou environ 0,1 à 10 % en poids/poids (p/p), ou environ 0,5 à 5 % en poids/poids (p/p) d'un ou plusieurs acides résiniques.

14. Utilisation selon l'une quelconque des revendications 8 à 13, dans laquelle la composition comprend en outre du glutaraldéhyde, dans laquelle la composition comprend facultativement environ 0,1 à 5 % en poids/poids (p/p) de glutaraldéhyde.
